(19) Europäisches Patentamt / European Patent Office / Office européen des brevets

(11) EP 2 173 855 B1

(12) # EUROPEAN PATENT SPECIFICATION

(45) Date of publication and mention of the grant of the patent:
**31.08.2016 Bulletin 2016/35**

(21) Application number: **08790064.3**

(22) Date of filing: **17.07.2008**

(51) Int Cl.:
***A23L 29/00*** *(2016.01)* ***A23L 33/175*** *(2016.01)*

(86) International application number:
**PCT/IT2008/000481**

(87) International publication number:
**WO 2009/011008 (22.01.2009 Gazette 2009/04)**

(54) **PROCESS FOR THE PREPARATION OF GAMMA-AMI NO BUTYRIC ACID (GABA) BY THE USE OF LACTIC ACID BACTERIA (LAB) ON AGRO- AND FOOD-INDUSTRY SURPLUS**

VERFAHREN ZUR HERSTELLUNG VON GAMMA-AMINOBUTYRINSÄURE DURCH VERWENDUNG VON ÜBERSCHÜSSIGEN MILCHSÄUREBAKTERIEN AUS DER LANDWIRTSCHAFT UND DER LEBENSMITTELINDUSTRIE

PROCEDE DE PREPARATION D'ACIDE GAMMA-AMINOBUTYRIQUE (GABA) AU MOYEN DE BACTERIES D'ACIDE LACTIQUE (LAB) SUR DES SURPLUS DE L'INDUSTRIE AGRO-ALIMENTAIRE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **17.07.2007 IT RM20070398**

(43) Date of publication of application:
**14.04.2010 Bulletin 2010/15**

(73) Proprietor: **Giuliani S.p.A.**
**20129 Milano (IT)**

(72) Inventors:
- **GIULIANI, Giammaria**
  **I-20129 Milano (IT)**
- **BENEDUSI, Anna**
  **I-20129 Milano (IT)**
- **DI CAGNO, Raffaella**
  **I-20129 Milano (IT)**
- **RIZZELLO, Carlo, Giuseppe**
  **I-70125 Bari (IT)**
- **DE ANGELIS, Maria**
  **I-70125 Bari (IT)**
- **MAZZACANE, Francesco**
  **I-70125 Bari (IT)**
- **GOBBETTI, Marco**
  **I-70125 Bari (IT)**

(74) Representative: **Gitto, Serena et al**
**Barzanò & Zanardo Roma S.p.A.**
**Via Piemonte, 26**
**00187 Roma (IT)**

(56) References cited:
**WO-A-2007/075011** **JP-A- 2002 360 289**
**JP-A- 2005 102 559**

Remarks:
The file contains technical information submitted after the application was filed and not included in this specification

EP 2 173 855 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> Giuliani S.p.A.

<120> Process for the preparation of gamma-aminobutyric acid (GABA) by the use of Lactic Acid Bacteria (LAB) on agro- and food-industry surplus

<130> PCT27328

<150> RM2007A000398
<151> 2007-07-17

<160> 3

<170> PatentIn version 3.5

<210> 1
<211> 496
<212> DNA
<213> Lactobacillus plantarum

<220>
<221> misc_feature
<222> (2)..(2)
<223> "n" is a or c or g or t/u

<220>
<221> misc_feature
<222> (4)..(4)
<223> "n" is a or c or g or t/u

<220>
<221> misc_feature
<222> (13)..(13)
<223> "n" is a or c or g or t/u

<220>
<221> misc_feature
<222> (19)..(19)
<223> "n" is a or c or g or t/u

<220>
<221> misc_feature
<222> (20)..(20)
<223> "n" is a or c or g or t/u

<400> 1

```
gnangctatt cangcctgnn cctggcaact ttttgtcaga cctatatgga acccgaagcc     60
gttgaattga tgaaggatac gctggctaag aatgccatcg acaaatctga gtacccccgc    120
acggccgaga ttgaaaatcg gtgtgtgaac attattgcca atctgtggca cgcacctgat    180
gacgaacact ttacgggtac ctctacgatt ggctcctctg aagcttgtat gttaggcggt    240
ttagcaatga aattcgcctg gcgtaaacgc gctcaagcgg caggtttaga tctgaatgcc    300
catcgaccta acctcgttat ttcggctggc tatcaagttt gctgggaaaa gttttgtgtc    360
tactgggacg ttgttttttt ttttttttcc caatggatga gcaacacgtg gcccttgggg    420
ttaaacacgt ctacggcttg ggccgagaat cgcttggtat ttttttgtaa acaaggcgtc    480
ccctttaccc ctgccc                                                    496
```

<210> 2
<211> 26
<212> DNA
<213> artificial

<220>
<223> GAD gene core forward primer

<400> 2
cctcgagaag ccgatcgctt agttcg 26

<210> 3
<211> 27
<212> DNA
<213> artificial

<220>
<223> GAD gene core reverse primer

<400> 3
tcatattgac cggtataagt gatgccc 27

## Description

This invention concerns a process for the preparation of gamma-aminobutyric acid (GABA) by means of Lactic Acid Bacteria (LAB) on agro- and food-industry surplus. In particular, this invention relates to the use of lactic bacteria which have been selected for the production of gamma-aminobutyric acid (GABA) on agro- and food-industry surplus like must for advantageous use in the dermatological field and in the treatment of kidney damages, for instance those which are caused by hypertension and by diabetic pathology. The GABA, defined as a non-natural amino acid, is synthesized by the enzyme glutamate decarboxylase (GAD) (EC 4.1.1.15), pyridoxal phosphate-dependent, that catalyzes the irreversible decarboxylation of L-glutamate in GABA. The enzyme GAD is widely distributed both in eukaryote and prokaryote organisms (Ueno, 2000, Enzymatic and structural aspect on decarboxylase, J. Mol. Catal. 10:67-79). It has been largely shown that the GABA can develop various physiological functions in Man. It can act as a neurotransmitter, as an hypotension inducing agent, diuretic and as a tranquillizer (Guin et al., 2003, GABA, gamma-hydroxybutyric acid, and neurological disease, Ann. Neur. 6:3-12; Jakobs et al., 1993, Inherited disorders of GABA metabolism, J. Inherit. Metab.Di 16:704-715*).* Treatments of depression (Okada et al., 2000, Effect of the defatted rice germ enriched with GABA for sleeplessness, depression, autonomic disorder by oral administration, Nippon Shokuhin Kagaku Kaishi 75:596-603), of alcoholism-connecetd symptoms (Oh et al., 2003, Germinated brown rice extract shows a nutraceutical effect in the recovery of chronic alcohol-related symptoms, J. Med. Food., 6:115-121) and the stimulation of the immune system (Oh and Oh, 2003, Brown rice extracts with enhanced levels of GABA stimulate immune cells, Food Sci. Biotechnol. 12:248-252) have been assosciated to the administration of GABA. Recent studies in the dermatological field have shown the involvement of GABA: (i) in homeostatic mechanisms at the epidermal level, as a response to UV radiations (Warskulat et al., 2004, The osmolyte strategy of normal human keratinocytes in mantaining cell homeostasis, J. Invest. Dermatol. 123:516-521); (ii) in the regulation of barrier receptors for preventing dysfunctions and hyperproliferative pathologies of epidermis (Denda et al., 2002, gamma-aminobutyric aid (A)receptor agonists accelerate cutaneous barrier recovery and prevent epidermal hyperplasia induced by barrier disruption, J. Invest. Dermatol. 119:1041-1047), and (iii) in stimulation of hyaluronic acid and in the improvement of survival of fibroblasts of epidermis following oxydative stresses (Ito et al., GABA-synthesizing enzyme, GAD67, from dermal fibroblasts: evidence for a new skin function, Biochim. Biophys. Acta, 1770:291-296). Moreover, the presence has been shown recently of numerous sub-units of the receptor for the GABA in kidneys, the assembling of sub-units into a new receptor complex and an active receptor for $GABA_A$ in kidneys proximal tubular cells (Satinder S.et al., The Journal of pharmacology and experimental therapeutics, vol. 324 no. 1; 376-382, 2008).

On the basis of such well recognized physiological functions, various functional food-stuffs have been enriched or strengthened with GABA by means of technological treatments, of an enzymatic or a microbial nature (Okada et al., 2000; Nagaoka, 2005, Treatment of germinated wheat to increase levels of GABA and IP6 catalyzed by endogenous ezymes, Biotechnol. Prog. 21: 405-410; Oh, 2003,Stimulation of gamma-aminobutyric acid synthesis activity in brown rice by a chitosan/Glu germination solution and calcium/calmodulin, J. Biochem. Mol. Biol. 36:319-325).

Some studies have also considered the synthesis of GABA from lactic bacteria (Nomura et al., 1998, Production of gamma-aminobutyric acid by cheese starters during cheese ripening, J. Dairy Sci. 81:1486-1491; Park and Oh, 2004, Cloning and expression of a full length glutamate decarboxylase gene from Lactobacillus plantarum, J. Food Sci. Nutr. 9:324-329:Park and Oh, 2007, Cloning, sequencing and expression of a novel glutamate decarboxylase gene from a newly isolated lactic acid bacterium, Lactobacillus brevis OPK-3, Bioresour. Technol. 98:312-319), both for the production of functional milk-cheese derivatives and for the understanding of the physiological mechanism which underlies such enzymatic activity. In general, the synthesis of GABA supplies bacterial cells subject to acid environmental conditions with resistance (Cotter and Hill, 2003, Surviving the acid text: responses of the of Gram-positive bacteria to low pH, Microbiol. MoLRev., 67:429-453) and the process exerted by the enzyme GAD has been associated with the synthesis of energy in Lactobacillus sp. E1 (Higuchi et al., 1997, Exchange of glutamate and gamma-aminobutyrate in a Lactobacillus strain, J. Bacteriol. 179:3362-3364). The production of GABA in the milk-cheese derivatives belonging to the published documents has not gone beyond the threshold of about 70 mg/liter (about 0.67 mM). At the present state of our knowledge also the patent JP-A-2005-102559 about the use of *Lactobacillus paracasei* NFRI (7415), isolated from the 'funa-zushi', for large scale production in culture soil MRS and during the preparation of a drink based on rice germs and bovine milk. In addition, the patent applications JP2002360289 and WO2007075011 disclose the preparation of GABA by means of lactic acid bacteria using natural substrates. Moreover, a study is known (Komatsuzaki et al., 2005, Production of gamma-aminobutyric acid (GABA) by Lactobacillus paracasei isolated from traditional fermented foodstuffs, Food Microbiol. 22:497-504) in which the production of GABA on culture soil MRS by means of the same biotype *L. paracasei* NFRI has been optimized with reference to some fermentation parameters. However, yields of GABA obtained by means of the procedures described in the known technique are not satisfactory as yet.

In the light of what has been described above it is evident thus that there is the need for materials and methods for the preparation of GABA which would be capable of overcoming the disadvantages of the procedures already known.

The authors of this invention have now selected some lactic bacteria, in particular *Lactobacillus plantarum* DSM 19463 (filed 26th June, 2007 with the DSMZ), substrates fit for their growth, in particular the must, and they have set forth a procedure for the production of GABA with yields much larger than those obtainable according to the technique already known.

Further advantages of this invention are: (i) the possibility of employing low-cost and natural fermentation substrates (for instance must) avoiding the use of commercial ad complicated culture soils in order to favour the growth of microorganisms and the biosynthesis of GABA; (ii) the growth of GABA synthesis on matrices of alimentary origin with respect to the concentrations reported in the literature; (iii) a fermentation procedure that allows a desiccated or lyophilized product to be obtained featuring a high phisiological value, naturally enriched with GABA, vitamins, minerals, polyphenols and alive aand vital lactic bacteria, which would be potentially probiotic; and (iv) the possibility of extending the field of application of the product based on GABA to the dermatological field and to the field of treatment of kidney damages.

Lactic bacteria according to the present invention belong to the genus of *Lactobacillus* and *Lactococcus.* They have been isolated previously from alimentary matrices (cheese) that had been naturally enriched with GABA and selected on the basis of their capability to synthesize high GABA concentrations in a culture soil. In the present invention the selection has been carried out on eight producers of GABA on the basis of their capability to develop and synthesize GABA starting from substrates like must, integrated with yeast water or with yeast extract, and fermented under standardized cultural conditions.

In particular, the *L. plantarum* DSM 19463 has been selected for fermentation of concentrated must (total amount of sugars 61.8%) diluted (total amount of sugars 1 %) in the ratio of 50:50 with yeast water and water, integrated with monosodium L-glutamate (20 mM) and modified as to its initial pH value of about 6.0.

A GABA production protocol from L. plantarum DSM 19463 has been standardized and optimized, said protocol providing its initial propagation in a culture soil MRS for 24 hours at 30°C, the collection of cells by centrifugation and washing, inoculation in diluted must with yeast water and integrated with 20 mM of monosodic L-glutamate, pH about 6.0, and the successive incubation preferably for a period of 72 - 78 hours at 30°C.

The fermentation process by means of *L. plantarum* DSM 19463 according to this invention allows (i) the exploitation of must, as a surplus coming from the food- and agro-alimentary field and thus substrates at zero cost; (ii) the production of about 500 mg/l (about 4.8 mM) of GABA at the end of the fermentation process of the must, or the production of about 890 mg of GABA per 100 g of a dry substance of the lyophilized product; (iii) the natural enrichment of the GABA-based product with vitamins of the group B that stem from the use of yeast water and with minerals and polyphenols stemming from the use of must; (iv) the natural enrichment of the lyophilized product with living and vital cells of *L. plantarum* DSM 19463 (about $10^{10}$ ufc/g under laboratory conditions) which on the basis of in vitro tests might feature any probiotic properties; and (v) the potential application of the product in the dermatological field, as is shown in the recent publication mentioned above.

*L. plantarum* DSM 19463 according to the present invention shows the production of GABA on a matrix of alimentary origin (must) of about 890 mg per 100 g of dry matter, which is much larger than that reported in the patent JP-A-2005-102559 (103,9 mg per 100 g of the dry matter) following the fermentation of a different alimentary matrix (a beverage based on rice germs and bovine milk) by means of *L. paracasei* NFRI (7415) isolated from 'funa-zushi'. With reference to L. plantarum DSM 19463 also the partial sequence of the GAD gene has been cloned, differently from the *L. paracasei* NFRI, the GAD gene being involved in the synthesis of GABA.

The partial sequence of the gene glutamate decarboxylase (GAD) of *L. plantarum* DSM 19463 (SEQ ID NO: 1) is the following:

1 gnangctatt cangcctgnn cctggcaact ttttgtcaga cctatatgga acccgaagcc

61 gttgaattga tgaaggatac gctggctaag aatgccatcg acaaatctga gtacccccgc

121 acggccgaga ttgaaaatcg gtgtgtgaac attattgcca atctgtggca cgcacctgat

181 gacgaacact ttacgggtac ctctacgatt ggctcctctg aagcttgtat gttaggcggt

241 ttagcaatga aattcgcctg gcgtaaacgc gctcaagcgg caggtttaga tctgaatgcc

301 catcgaccta acctcgttat ttcggctggc tatcaagttt gctgggaaaa gttttgtgtc

361 tactgggacg ttgttttttt ttttttttcc caatggatga gcaacacgtg gcccttgggg

421 ttaaacacgt ctacggcttg ggccgagaat cgcttggtat ttttttgtaa acaaggcgtc

481 ccctttaccc ctgccc

The authors of the present invention have also selected the lactic bacterium *Lactococcus lactis* ssp. DSM 19464 (filed on 26th June, 2007 with the DSMZ) that can be employed in the fermentation of must according to a fermentation protocol with different GABA yields.

Accordingly, it is a specific object of the present invention the *L. plantarum* DSM 19463 or the Lc. lactis ssp. DSM 19464 or their associations.

It is a further object of the present invention the use of *L. plantarum* DSM 19463 or the *Lc. lactis* ssp. DSM 19464 or their associations for the preparation of a probiotic mixture enriched with gamma-aminobutyric acid.

Moreover, this invention also relates to a process for the preparation of a probiotic mixture enriched with gamma-aminobutyric acid that includes the following steps:

a) propagation on a culture soil, preferably MRS or M17, of *L. plantarum* DSM 19463 or *Lc. lactis* ssp. DSM 19464 or their associations for 24 hours at 30°C;

b) resuspension of the so obtained Biomass in step a) in a part of a substrate added with 20 mM monosodic L-glutamate, said substrate being must according to the cell density of about $10^9$ ufc/g;

c) inoculation of the substrates at the percentage of 1-4% and fermentation for 72-96 hours, preferably 72-78 hours, at the temperature of 30-33°C, and preferably at 30°C.

Preferably, the must can be diluted with yeast water or it can be added with yeast. In particular, must can be diluted with yeast water and 50/50 distilled water, and can be deacidified by means of NaOH 1 N down to a pH value of 6.0 -6.5, preferably, about 6.0. Moreover, must can be added with pyridoxal 5-phosphate.

The process according to the present invention can further include a step d) of desiccation or lyophilization of the fermentation substrate obtained in step c).

Accordingly, on the basis of what has already been shown above, the process for preparing a probiotic mixture enriched with gamma-aminobutyric acid will include the following steps:

a) propagation on culture medium, preferably MRS or M17, of *L. plantarum* DSM 19463 or *Lc. lactis* ssp. DSM 19464 for 24 hours at 30°C;

a1) collection of of cells by means of centrifugation (10,000 x g for 10 minutes at 4°C), washing by means of phosphate 50mM buffer, pH 7,0, and

b) resuspension of the Biomass in a portion of diluted must according to a cell density of about $10^9$ ufc/g;

b1) preparation of yeast water by suspending about 60 g commercial brewer's yeast in about 300 ml distilled water; sterilization in autoclave at 120°C for 30 minutes; decantation for 12 hours at 4°C of the Biomass; centrifugation at 6,000 x g for 10 minutes at 4°C; and final recovery under sterile conditions of the supernatant (yeast water) containing the cytoplasmic extract of yeast cells;

b2) dilution of the concentrated must with water and yeast water according to the proportions already described above (final pH of about 6,0 after modification) and successive sterilization in autoclave at 120°C for 15 minutes,

c) inoculation of the substrates at 1 - 4% and fermentation for about 72 - 96 hours (preferably 72 - 78 hours) at 30°C.

The process according to the present invention can further include a step d) which is a desiccation or lyophilization step of the fermentation substrate obtained in step c).

It is a further object of this invention a probiotic mixture enriched with gamma-aminobutyric acid that can be obtained by means of the process that has been set forth above, said mixture comprising *Lactobacillus plantarum* DSM 19463 or *Lactococcus lactis* ssp. DSM 19464 or their associations, and preferably the mixture contains at least 890 mg of gamma-aminobutyric acid per 100 g of dry substance, and in addition it includes vitamins, minerals, polyphenols and live and vital lactic bacteria.

The probiotic mixture can be employed advantageously in the medical and the cosmetical fields and, according to studies carried out by the applicant and reported in the experimental part of this patent application has shown advantageous effects with respect to those obtained following the treatment with the GABA only. Accordingly, this invention will also concern a pharmaceutical or cosmetic composition comprising a probiotic mixture enriched with gamma-aminobutyric acid as already described above, as the active principle, together with one or more excipients and/or adjuvants as pharmaceutically or cosmetically acceptable compounds.

It is a further object of this invention the use of the probiotic mixture enriched with gamma-aminobutyric acid as a cosmetic compound according to the present invention.

Moreover, this invention relates to the use of the probiotic mixture enriched with gamma-aminobutyric acid according to the present invention for te preparation of a medicament for dermatological use.

It is a further object of the present invention the pharmaceutical composition as is defined above for treating kidney diseases as for instance those renal damages which stem from hypertension and from diabetic pathology.

This invention will be disclosed now just for illustration and not for limitative purposes according to its preferred forms of realization and with particular reference to the figures of the enclosed drawings, in which:

figure 1 shows the acidification kinetics and the growth of *L. paracasei* $FC_4$4, *Lactobacillus plantarum* $FC_4$15, *Lactobacillus delbrueckii* PR1, *L. plantarum* $FC_4$8 and *L. lactis* ssp. DSM 19464 on diluted must (total sugars 1%) with distilled water, pH about 4,0. The fermentation has been carried out at 30°C;

figure 2 shows the acidification kinetics and the growth of *L. paracasei* $FC_4$4, *L. plantarum* $FC_4$15, *L. delbrueckii* PR1, *L. plantarum* DSM 19463, *L. plantarum* $FC_4$8 and *Lc. lactis* ss. DSM 19464 on diluted must (total sugars 1 %) with yeast water and distilled water in the ratio of 50:50, pH about 4,5. The fermentation has been carried out at 30°C;

figure 3 shows the production of gamma-aminobutyric acid (GABA) as mg/l from *L. plantarum* DSM 19463 in diluted must (total sugars 1 %) with yeast water and distilled water (50/50) at pH about 4,5, and added with monosodic L-glutamate (20mM) (A); water diluted must (total sugars 1 %), pH about 4,5, and added with yeast extract (0.5%) and monosodic L-glutamate (20 mM)(B); buttermilk, pH 5,90, added with monosodic L-glutamate (20mM) (C); and buttermilk, pH 5,90, added with yeast extract (0,5%) and monosodic L-glutamate (20mM)(D). The fermentation has been carried out at 30°C;

figure 4 shows the acidification kinetics (A) and the growth (B), and the production of gamma-aminobutyric acid (GABA) as mg/l from *L. plantarum* DSM 19463 (C) in diluted must (total sugars 1 %) with yeast water and distilled water (50/50, pH about 6,0 and added with monosodic L-glutamate (20mM). The fermentation has been carried out at 30°C;

figure 5 shows the production of gamma-aminobutyric acid (GABA) as mg/l from *L. plantarum* DSM 19463 (C) in diluted must with different percentages of total sugars (0.3%, A; 0.5%, B; 0.7%, C; and 1%, D) with yeast water and distilled water (50/50), pH about 6,0, and added with monosodic L-glutamate (20mM). The fermentation has been carried out at 30°C;

figure 6 shows the acidification kinetics (A) and the growth (B), and the production of gamma-aminobutyric acid (GABA) as mg/l from *L. plantarum* DSM 19463 (C) in diluted must (total sugars 1%) with yeast water and distilled water (50/50, pH about 6,0 and added with monosodic L-glutamate (20mM) and pyridoxal 5-phosphate. The fermentation has been carried out at 30°C;

figure 7 shows the acidification kinetics (A) and the production of gamma-aminobutyric acid (GABA) as mg/l from resting cells (cells not in the growth phase) of *L. plantarum* DSM 19463 (B) in diluted must (total sugars 1%) with yeast water and distilled water (50/50), pH about 6,0 and added with monosodic L-glutamate (20mM). The fermentation has been carried out at 30°C;

figure 8 shows the productivity as $gl^{-1}h^{-1}$ (A), yeald as $\Delta P/\Delta S$ (B) and the specific production rate as 1/x dP/dt (C) of gamma-aminobutyric acid (GABA) from *L. plantarum* DSM 19463 in diluted must (total sugars 1%) with yeast water and distilled water (50/50), pH about 6,0, and added with monosodic L-glutamate (20mM). The fermentation has been carried out at 30°C;

figure 9 shows the expression of the enzyme hyaluronate synthetase in an FT-skin model following the treatment at 8, 24, 48 and 72 hours with the Biomass according to the present invention or GABA;

figure 10 shows the expression of the gene for the philagrin in an FT-skin model following the treatment at 8, 24, 48 and 72 hours with the Biomass according to the present invention or GABA;

figure 11 shows the expression of the gene for the involucrin in an FT-skin model following the treatment at 8, 24, 48 and 72 hours with the Biomass according to the present invention or GABA.

Example 1: *Selection of lactic bacteria according to the present invention*

Lactic bacteria belonging to the Collection of Cultures of the Department of Protection of Plants and Applied Microbiology of the University of Bari, Italy (Collezione di Colture del Dipartimento di protezione delle Piante e Microbiologia Applicata dell'Universita' degli Studi di Bari), previously isolated from cheese and selected on the basis of their capability of synthesizing GABA under conditions of culture medium, have been cultivated at 30°C for 24 hours in MRS or in M17 (*Lc. lactis* ssp. DSM 19464) (Oxoid, Basingstoke, Hampshire, England). In Table 1 the list is reported of the species and biotypes employed in the present invention for the production of gamma-aminobutyric acid.

Table 1

| Species | Strain |
|---|---|
| *Lactobacillus paracasei* | $FC_4$4; $FC_4$10 |
| *Lactobacillus plantarum* | DSM 19463; $FC_4$4; $FC_4$12; $FC_4$15 |
| *Lactobacillus lactis* ssp. | DSM 19464 |
| *Lactobacillus delbrueckii* | *PR1* |

**(1) Using lactic bacteria for fermentation of agro- and food-matrices**

After cultivation on culture medium, lactic bacterial cells have been collected by means of centrifugation (10,000 g x 10 min., 4°C), then they have been washed twice in a 50mM phosphate buffer, pH 7,0, and resuspended in a portion of must or of buttermilk, at the cell density of 109 ufc/ml.

Concentrated must (sugar total amount 59.2%) not added with sulphurous anhydride, was diluted to the concentration of total sugar of 1 % with distilled water, or with distilled water and yeast water in the ratio of 50/50, then it was deacidified by means of NaOH 1 N at a pH of about 4,0 - 4,5 or about 6,0 and then sterilized in autoclave at 120°C for 15 minutes. When yeast water is employed, it had been prepared by suspending about 60 g commercial brewer's yeast in about 300 ml distilled water; sterilizing in autoclave at 120°C for about 30 minutes; separing by decantation for 12 hours at 4°C the Biomass; then centrifuging at 6,000 x g for 10 minutes at 4°C; and final recovery under sterile conditions of the supernatant (yeast water) containing the cytoplasmic extract of yeast cells.

The buttermilk, obtained as a side product of the production of 'ricotta' has been characterized by the following parameters: lactose 4.80%, proteins 0.79%, fats 0.40%, pH 5,0. Said buttermilk has been employed in fermentation processes as such or with the addition of a yeast extract (0.5%).

Monosodic L-glutamate 20mM was added to the fermentation substrates and, in some experiments, pyridoxal 5-phosphate 0.1 mM was added.

Fermentations were carried out by means of inoculum of 4% lactic bacteria (initial cell density of about $10^7$ ufc/ml) and incubation at 30°C for 96 hours.

When 'resting cells', i.e. cells not in the growth phase, are employed, an inoculum was provided capable of allowing an initial cell density equal to about 10 ufc/ml to be obtained. When *L. plantarum* DSM 19463 and *Lc. lactis* ssp. DSM 19464 have been used in association, they have been inoculated according to a cell density of about $10^7$ ufc/ml each.

**(2) Growth kinetics and acidification**

Cell density of lactic bacteria has been determined by MRS agar plate counting (Oxoid). Acidification kinetics of the kneaded soft mixture was obtained on line by means of the measurement of pH (pH-meter 507, Crison, Italy). Data relating to the growth and acidification kinetics have been modelled through Gompertz equation modified by Zwietering et al. (Zwietering et al., 1990. Modelling of bacterial growth curve. Appl. Environ. Microbiol. 56: 1875-1881). As to the initial experiments, the growth of lactic bacteria on different substrates was tested by the determination of the optical density at 620 nm (O.D.$_{620}$). The determination of the D- and L-lactic acid was carried out by means of an enzymic kit (DHFF CHAMB Italia Srl, Italy).

**(3) Determination of GABA**

GABA concentration in the different fermented substrates was determined by means of the 'Amino Acid Analyzer Biochrom 30' (Biochrom Ltd., Cambridge, UK) employing a cationic exchange column (Na Oxidised Feedstuff, 20 cm x 4.6 mm) (Di Cagno et al., 2007, Characterization of Italian Cheeses Ripened Under Nonconventional Conditions, J. Dairy Sci. 90: 2689-2704).

The production of GABA was also characterized in terms of productivity as $gl^{-1}h^{-1}$; yield as $\Delta P/\Delta S$, i.e. production of GABA with respect to the consumption of the substrate (monosodic L-glutamate); and production specific rate (1/x dP/dt), where x represents the cell density at the time considered.

**(4) Determination of the content of vitamins, minerals and polyphenols**

The content of B group vitamins and of polyphenols of the fermented substrates was deter-mined by means of HPLC analysis. The concentration of minerals was determined by the atomic absorption method.

(5) Molecular characterization of the GAD gene in *Lactobacterium plantarum* DSM 19463

Extraction of DNA from *L. plantarum* DSM 19463 was carried out by means of the method reported by De Los Reyes-Gavilan (De Los Reyes-Gavilan et al., A Lactobacillus helveticus-specific DNA probe detects restriction fragment length polymorphisms in this species. Appl. Environ. Microbiol. 58:3492-3432). Prmers designed by highly conserved regions of the GAD gene, CoreF/CoreR (5'-CCTCGAGAAGCCGATCGCTTAGTTCG-3 (SEQ. ID NO2) and 5'-TCATATTGACCGGTATAAGTGATGCCC-3' (SEQ ID NO 3)) (PRIM srl San Raffaele Biomedical Science Park, Milano, Italy) have been used for amplifying the gene of GAD. Fifty microliters of each reaction of PCR (Polymerase Chain Reaction) contained: 200 μM of each 2'-desoxyribonucleotide 5'-triphosphate, 1 μM of each primer, 2 μM of MgCl, 2 U of Taq DNA polymerase an about 50 ng of DNA. PCR amplification reactions were carried out by means of the GeneAmpPCR System 9700 (Applied Biosystem, USA). The amplification products were separated by means of electrophoresis on 1.5% agarose gel and coloration was obtained by means of ethidium bromide (0.5 μg/ml). Amplicons eluted by means of the gel were purified through GFX PCR DNA and Gel Band Purification Kit (Amersham Biosciences, Upsala, Sweden). Sequencing recations of DNA have been carried out by PRIM. Sequence comparison was carried out by means of a Basic BLAST database. Translation of the nucleotide sequences was carried out by means of the OMIGA software (Oxford Molecular, Madison, USA).

**Results**

**(1) Growth and acidification on must and buttermilk**

Considering the biotechnological innovation connected to the use of must and buttermilk as substrates for the synthesis of GABA starting from lactic bacteria, the potentiality of growth and acidification of the bacteria was initially tested on the two substrates. As is possible to observe in Fig. 1, also the distilled-water diluted must (total sugars 1%), pH modified to about 4,0, allowed a moderate growth of lactic bacteria. Examples concerning the cultures reported in Fig. 1 showed an $OD_{620}$ value of 0.89 - 1.06 after 48 hours incubation at 30°C. Also the other bacteria employed for the initial screeing showed $OD_{620}$ values in the range reported above. To the microbial growth various pH values in the range from 3,11 to 3,65 corresponded (Fig. 1). When the must dilution was carried out not ust by means of distilled water but also with yeast water (ratio of 50:50) microbial growth was definitely increased (Fi. 2). As was estimated by means of the determination of $OD_{620}$, bacterial growth was shown to be in the range from 2.28 to 2.48, with the highest value recorded in the case of L. plantarum DSM 19463. The same results were also obtained by comparing the lactic bacteria growth on buttermilk as such or on buttermilk added with yeast extract (0.5%). In particular, Lc. *Lactis* ssp. DSM 19464 has shown the best growth on buttermilk. At the end of this first step of selection it was thus decided to go on with the experimental part on *L. plantarum* DSM 19463 and on *Lc. lactis* DSM 19464 employing as substrates must diluted (total sugars 1%) with yeast water and distilled water (in the ratio of 50:50), and buttermilk added with yeast extract (0.5%).

(2) Synthesis of GABA from Lactobacillus plantarum DSM 19463 and *Lactococcus lactis* ssp. DSM 19464

Fig. 3 shows the synthesis of GABA from *Lactobacillus plantarum* DSM 19463 on must and buttermilk integrated differently. A further modification that concerns the use of must (pH of about 4,5) was the addition of monosodic L-glutamate (20 mM) as a substrate for the enzyme GAD activity. The pH value of the buttermilk was obviously of about 5,9. As is observed in the case of growth and acidification processes, the addition of yeast water favoured the highest productions of GABA, which productions after 96 hours of fermentation became fixed at values of about 95 g/l (about 0.91 mM). GABA productions on buttermilk were shown to be remarkably lower, even in the presence of yeast extract.

On the contrary, *Lc. lactis* ssp. DSM 19464 showed the highest synthesis of GABA on buttermilk to become fixed at values of about 80 g/l (0.77 mM).

On the basis of the results so obtained it was decided to anyway optimize the production of GABA from *L. plantarum* DSM 19463 using must as the only substrate.

(3) Optimization of the synthesis of GABA from *L. plantarum* DSM 19463 on must

In particular the following fermentation variables have been considered: (i) pH; (ii) initial concentrations of sugars in the must; (iii) addition of pyridoxal 5-phosphate as a co-factor of the enzyme GAD; (iv) use of 'resting cells'; and (v) combined use of *L. plantarum* DSM 19463 and *Lc. lactis* ssp. DSM 19464.

The use of a phosphate buffer in order to keep the pH value constant during the period of incubation forced the emplymemt of too high molarities, which turned out to be inhibitory of growth and enzymic activity of *L. plantarum* DSM 19463. With the aim at avoiding that the pH value would reach remarkably acid values too rapily during the incubation period, the must initial value of the pH was modified till the value of about 6,0, by adding NaOH 1 N. Figure 4 shows the acidification, growth and synthesis kinetics of GABA from L. plantarum DSM 19463 on diluted must (1% total sugars) with distilled water and yeast water, pH of about 6,0, and with the addition of monosodic L-glutamate (20 mM). It is possible to observe that in such conditions a strong acidification process char-acterized by values of $\Delta$pH = 2,12 units, $V_{max}$ = 0.151 dpH $min^{-1}$ and $\lambda$ = 0.89 h, to which the synthesis of about 25 mM of lactic acid corresponds. A microbial growth corresponds to such acidification kinetics, said growth being characterized ba values of A = 1.79 log cfu $ml^{-1}$, $\mu_{max}$ = 0.31 $\Delta$log cfu $ml^{-1}$ $h^{-1}$ and $\lambda$ = 0.89 h; and above all a constant increase of GABA synthesis that becomes fixed at the value of about 500 mg/ml (about 4.79 mM) after 78 hours of fermentation.

The concentration of initial sugars in the must was changed in the range from 0.3 to 1.0% (Fig. 5). Though the GABA synthesis is linked to catabolism reactions of amino acids, such synthesis has turned out to increase proportionally (376 - 500 mg/l) to the concentration of sugars (0.3 - 1%). Concen-trations higher than 1% didn't give meaningful increases in the synthesis of GABA. In the presence of initial concentration of 1%, just traces (< 0.1%) of glucose and fructose have been revealed in the must after 72-96 hours fermentation.

As is stressed in Fig. 6, the addition of pyridoxal 5-phosphate (0.1 mM) didn't favour an increase in the synthesis of GABA with respect to what is observed in the case of its absence (Figure 5). Evidently, the concentration of such compound in must is sufficient to develop the co-factor function of the enzyme GAD. As expected, also the acidi-fication and growth kinetics were not affected by the addition of pyridoxal 5-phosphate.

As the synthesis of GABA is a purely enzymic reaction that is not linked theoretically to a bacterial multiplication, the possibility has been evaluated of inoculating the must with a high Biomass (10 ufc/ml) of *L. plantarum* DSM 19463 under virtual conditions of 'resting cells'. Figure 7 shows that under such conditions of cell undevelopment the synthesis of GABA is lower than that observed in conditions of cell growth (Figure 5). The use of cells of *L. plantarum* DSM 19463 in such conditions just allowed a low increase to be attained in the GABA production during the first hours of incubation.

Again with the aim of increasing the production of GABA obtained from *L. plantarum* according to the fermentation conditions of Figure 5, the hypothesis has been considered of performing a combined inoculum of such bacterium in association with *Lc. lactis* DSM 19464. Also under such experimental conditions no increases were observed in the synthesis of GABA.

**(4) Characterization of the process of synthesis of GABA from L. plantarum DSM 19463 on must.**

Some biotechnological parameters like productivity as $gl^{-1}h^{-1}$, yield as $\Delta P/\Delta S$ and specific production rate (1/x dP/dt) of GABA have been determined during fermentation of must diluted with yeast water and distilled water (50/50 (total sugars 1 %), pH of about 6,0, and added with monosodic L-glutamate (20 mM), due to the action of *L. plantarum* DSM 19463. As is possible to observe in Figure 8, the higher values of productivity that were equal to 5.74 - 5.79 $gl^{-1}h^{-1}$ have been observed respectively after 72 - 78 hours. Accordingly, also the higher values of yield and specific rate of GABA, respectively of 0.206 and 0.673 1/x dP/dt have been observed after 72 hours of fermentation at 30°C.

After 72 hours of fermentation the culture broth was recovered and subjected as such to the lyophilization process. Table 2 reports the composition as to functional molecules and as to lactic bacteria of the preparation, in particular the concentration of functional molecules per 100 g of dry substance of the preparation which is based on must after fermentation by means of *L. plantarum* DSM 19463 and successive lyophilization.

Table 2

| Compound | Concentration |
|---|---|
| gamma-aminobutyric acid | 890 mg/100 g dry substance |
| Vitamin B3 | 25.8 mg/100 g |
| Total polyphenols (spectrophotometric method) | 2.09 g/100 g |
| Zinc | 28.1 mg/100 g |
| Copper | 1.11 mg/100 g |
| Magnesium | 155 mg/100 g |
| Lactic bacteria | $10^{10}$ ufc/g |

As is possible to observe from Table 2, the preparation contains a GABA concentration of about 890 mg/100 g of dry substance, the latter being more than eight times higher than that reported in the patent JP-A-2005-102559. The preparation contains also other functional molecules such as vitamins of the group B that derive from the use of yeast water as the diluting means for must, and minerals and polyphenols that stem from must as the basic substrate. Finally, about 10 cells per gram of *L. plantarum* DSM 19463 are present in the preparation, such cells being likely to feature potential probiotic activities on the basis of preliminary tests.

Example 2: Evaluating the efficiency of the mixture enriched with GABA according to the present invention and defined here as the Biomass or the bio-complex, on a model of human epidermis reconstituted in vitro and next on a model of skin FT reconstituted in vitro

This study was carried out in order to check the possible application of the mixture according to the pesent invention in case of atopic xerosis, seborrhoic dermatitis of skin and of scalp, improvement in the barrier function and in the action on the microflora.

In particular, the object of the present invention is first of all the checking of cutaneous tolerance of said BIOMASS after single aplications for different contact times on human epidermis reconstituted in vitro and the study of its interaction with vital epidermis and on the epidermic barrier function.

Then, in a second approach, the confirmation of the efficiency for the GABA according to the previous scientific literature, and of the action mechanism of Biomass with respect to GABA which is employed as the positive control on the following parameters.

**Experimental design**

**Test system: HUMAN reconstituted EPIDERMIS**

**1)** The epidermis model employed is produced by the laboratories Skinethic®, Nice (F) and is employed in the 0.5 cm size from the seventeenth day of differentiation with an average thickness of the lot of 120 μ (horny layer and vital epidermis).

A fully differentiated epidermis is obtained starting from human keratinocytes in a chemically definite culture medium (MCDB 153) without any addition of bovine foetal serum, on an inert support made up of porous polycarbonate at the air-liquid interface for 17 days; at that differentiation stage the morphological analysis shows a multi-layered vital epidermis and a horny layer formed by more than 10 compact celular layers.

2) The 'full thickness skin model' (FT) (Phenion GmbH & Co., Frankfurt am Main, Germany) is a multi-layer human skin model consisting of keratinocytes and fibroblasts stemming from the same donor. After a culture period of five weeks, the 'full thickness' was fully developed and comprised epidermis, basal membrane and derma. The model was characterized with respect to the expression of fundamental markers of differentiation at the epidermis level (cytokeratin 10, philagrin, transglutaminase and involucrin), at the derma-epidermis junctions (laminin and collagen IV) and at dermal level (elastin fibronectin). The diameter of the model FT is of 1.3 cm.

Media and culture conditions

Tissues and culture media are produced according to GMP and their reproducibility with respect to specific standards is ensured. The use of the same is authorized just for research purposes, for the in vitro evaluation of products or chemical substances.

The tissue lot was analyzed in order to guarantee the absence of HIV virus, of hepatitis B virus, of hepatitis C virus, of cytomegalovirus, of mycoplasma. Culture media were warranted to be sterile. The RHE inserts are sent on the seventeenth day of culture within wells containing a nutritional gel of agarose, in plates of 24 wells.

Immediately after their arrival in the laboratory the RHE are separated from the agarose gel under a laminar flow hood. The inserts were rapidly put in Falcon plates having 6 or 12 wells containing respectively 500 μl or 1 ml of maintenance medium SkinEthic at the room temperature. Care is taken in order to avoid the formation of bubbles under the insert. Tissues are placed in an incubator at 37°C in the presence of 5% $CO_2$ in an atmosphere saturated with humidity. Soil is changed at 24 hours intervals and always at least two hours before starting the test.

After arrival, the FT-skin tissue is immediately removed from the transportation semi-solid soil.

Three small Petri dishes without cover are placed in a 100 mm Petri dish and 5 ml of ALI-medium are distributed inside each one of them together with two metal supports an a small filter-paper disk on which the FT tissues are placed carefully.

The tissues are than put into an incubator (37°C, 5% $CO_2$ and 90% humidity).

Products

| name | lot | identification code | conservation | manufacturer |
|---|---|---|---|---|
| lyophilized Biomass | L2- | A74 | +4°C | Giuliani S.p.A. |
| Lb. plantarum | 00907 | | | |
| GABA | | GABA | | SIGMA |

Control samples

| name | lot | identification code | conservation | manufacturer |
|---|---|---|---|---|
| PBS | 79382 | A78CN | R.T. | Fluka |
| ISOCIDE PN | PN 01BX6 | A77CP | R.T. | Biophil |

**METHODS**

**MEASUREMENT OF THE TEER**

TEER (trans-epitelial electrical resistance) is a direct measure of functionality of the cutaneous barrier; it reflects all the global resistance of the tissue which is given by its own thickness as well as by its own structure. It reflects the integrality of intercellular contacts at the level of tight junctions, of the bilaminar lipidic structure that oppose penetration of outside compounds.

TEER is the discriminating parameter of the *rat skin electrical resistance* test (**B 40**) that is EU validated for evaluating corrosivity taking as the end-points the integrity of the horny layer and the barrier function.

TEER is inversely proportional to TEWL measured in vivo which is the measurement of the trans-epidermal water loss; the higher the TEWL, the higher the damage to the barrier function, whereas the higher the TEER, the lower the damage to the barrier function.

1 ml of PBS is placed on the insert and the trans-epitelial electrical resistance is measured with the Millicell-ERS instrument (range 0 - 20 kΩ).

Two measurements were carried out per each tissue.

**CYTOTOXICITY TEST: MTT TEST**

This method allows the quantification of a cytotoxic effect induced by the product by means of the measurement of cellular vitality. Cytotoxicity is quantified by measuring the decrease of cellular vitality with respect to a negative check of an untreated sample. The test is based on the metabolization reaction that occurs between the tetrazolium salt (MTT) and the mitochondrial enzymes (succinate dehydrogenase) after contact with the product for the different treatment times: the vital cell only can transform the tetrazolium salt into the insoluble derivative (formazane), a reaction that can be put into evidence by a violet coloration at the basis of the insert.

The metabolization of the salt occurs at the epitelium basal cells in the zone that is over the polycarbonate support. The extraction of the derivative which has a violet color is carried out in isopropanol and the quantification is carried out by spectrophotometric methods at 570 nm.

**Materials**

MTT tetrazolium salt (Sigma M2128) (Sigma L3771)
Saline solution (FU)
Isopropanol (Sigma)
Maintanance Medium (SkinEthic)
Microplate Autoreader M-200 INFINITE (TECAN)
Analytical Balance XS204 Mettler (0.00001)
Protocol

When the treatment time is over, the surfaces of the epidermises treated are washed with PBS (three times) and then transferred into a plate containing 300 μl of a solution of MTT (0.5 mg/ml in the medium).

After incubation for three hours at 37°C the formazane is extracted for two hours at the room temperature and under light agitation. The value of the O.D. is determined by spectrophotometric methods at 570m against isopropanol as the blank.

The percentage of cytotoxicity is calculated with respect to the values of the O.D. of the negative check sample.

**HISTOLOGICAL ANALYSIS**

Histological preparations necessary for histological analyses of the RHE are carried out by an outside specialized centre.

At the end of all treatments the RHE's are removed from their plastic support by means of a lancet and subjected to a fixing process employing 10% formalin.

**REAL TIME PCR**

In this study the following genes were employed:

| | |
|---|---|
| **DEFB4** | Hs00175474 m1 |
| **IL10** | Hs00174086 m1 |
| **IL12B** | Hs00233688 m1 |
| **HAS1** | Hs00155410 m1 |
| **GCLC** | Hs00155249 m1 |
| **GSS** | Hs00609286 m1 |
| **IVL** | Hs00846307 s1 |
| **FLG** | Hs00863478 g1 |

1. Extraction of RNA
2. Synthesis of cDNA starting from total RNA
3. PCR

The technique employs Taqman probes for detecting the fluorescent signal and it is very sensitive, consisting in the amplification and quantification of a specific sequence of nucleic acids. The detection of the PCR product occurs in real time. The quantification of DNA, cDNA or RNA is carried out by determining the cycle in which he PCR product can be detected for the first time, thus not at the moment when the reaction has become saturated. The quantification of the product occurs through the detection of fluorescence measured in each cycle; the strength of the signal is directly proportional to the amount of the amplified product.

**EXTRACTION OF RNA**

Materials

RNAqueous kit (AMBION)

## THE PRINCIPLE OF THE METHOD

RNAqueous is a rapid method for isolating filter-based RNA.The method is based on the capability of glass fibers to link the nucleic acids in concentrated sal solutions.
The concentration of RNA is determined by reading the value of the absorbance at 200 nm and 280 nm on a 96-well plate which was UV transparent and employing the following formula:

$$A260*dilution\ factor*40 = g\ RNA/ml$$

## Retrotranscription of RNA into cDNA

### Materials

High Capacity cDNA Reverse Transcription kit

(Applied Biosystems)

### The principle of the method

The principle of the method is based on the use of random primers that ensure an efficient synthesis of the first helix of all the RNA molecules present.
cDNA is diluted and conserved at -20°C.

## REAL TIME PCR

The quantification determines the change of expression of a sequence of nucleic acids (target) in a sample with respect to the same sequence in a calibrated sample that can an untreated control sample or sample at the reference time.
In the relative quantification an endogenous control is employed as the 'active reference' in order to normalize the cNA target.

### Materials

2X TaqMan Universal pcr Master Mix
20X TaqMan gene expression assay (Applied Biosystems);

### GAPDH (endogenous control) Hs 999999 m1

gene target, see the Table at 4.4
'nuclease free' water

### Procedure

Each biological sample is evaluated in triple tests. The thermocyclicator Applied Biosystems 7500 is employed by applying the followin program:

| Coditions | STEP 1. AmpliTaq Gold DNA Polymerase Activation | STEP 2: 40 cycles |
|---|---|---|
| TEMPERATURE | 95°C | 95°C 60°C |
| TIME | 10 MIN | 15 SEC 1 MIN |

The general data from the instrument are recorded by the internal SDS 1.3.1. software and reported as 'raw data' of relative quantification. The results are exported as Excel.
A value is accepted as meaningful if it is overexpressed or downregulated by one time with respect to the calibrating sample.

**PROTOCOLS**

**CUTANEOUS TOLERABILITY and efficiency of the**

**BARRIER FUNCTION**

**THE IRRITATING POTENTIAL** is evaluated on the model of epidermis SkinEthic after the acute application of the Biomass in the amount of 50 l (1% - 3% - 3% + preserving agent 0.8%) for four times: **24 hours - 24 hours - 24 hours recovery - 48 hours - 72 hours.**

Three complementary parameters have been studied with the aim of defining a global and predictive evaluation of the biological action of the Biomass at the cutaneous level as that type of evaluation has never been realized as yet and it represents a new way of research in dermatology. The evaluation allows the monitoring of tolerability to be carried out and at the same time the monitoring of the biological action described in the literature by means of specific biomarkers.

Monitoring of the cell vitality through the MTT test whic defines the potential of cutaneous irritation: in that case we wanted to check not only the result after near-term acute application (24 hours) but also the long-term effects considering the potential biological activity of the Biomass.

Histological evaluation H&E, complementary and for confirmation of the cytotoxicity values.

Apart from the interest for the antimicrobial action and thus for the increase in the physiological antimicrobial protection, the beta-defensins have a role of global regulation of the innate immunity at the cutaneous level because they increase the cell proliferation and the production of specific cytokines.

**ACTION ON THE SYNTHESIS OF HYALURONIC ACID ON THE REDOX EQUILIBRIUM and on markers of terminal differentiation (filagrin and involucrin) on the FT-skin model with respect to GABA**

The products (1% Biomass and GABA) are applied in amounts of 50 l on the surface of the tissue and then applied again every day for 3 days = 72 hours. At the end of the treatment the tissues are processed for the RT-PCR.

This type of treatment in the absence of negative stimuli and on a tissue in a 'physiological' situation allows the biological effects of a repeated exposition to the ingredient and of its mechanism of action to be put into evidence at the dermal level.

The positive GABA control applied to the 1 mm dose has been chosen because its efficiency is documented in the literature both on the synthesis of hyaluronic acid that in the protection against the oxidative stress through the increased production of GSH, a hydrophilic non-enzymic antioxidant.

**DATA ACQUISITION**

The general data obtained by means of the instrument ABI PRISM 7500 are recorded by the internal SDS 1.3.1. software and they are reported as 'raw data' of relative quantification. The results are exported as Excel.

A value is accepted as meaningful if it is overexpressed or is down-regulated by one time with respect to the calibrating sample.

The histological samples (slide glasses) are analyzed by means of the microscoy Leica DM2500, the images are saved and processed by means of the LEICA APPLICATION SUITE (LAS) software.

The morphological modifications of the tissues are compared to the untreated tissues.

RESULTS

CUTANEOUS TOLERABILITY

The results of the MTT test expressed as % cellular VITALITY calculated with respect to the negative control sample. The results of the MTT test at all times defined show that no cytotoxic activity can be attributed to the compound under examination, i.e. the Biomass from LB plantarum.

The presence of an antimicrobial preserving agent (isocide 0.8%) didn't affect the results of cytotoxicity.

It is stressed that the cellular vitality after treatment with the Biomass both at 1% and at 3% increased (at the times 24 hours + recovery and 48 hours) beyond the value of intrinsic variability of the test (+/- 15%)with an increase of 40% with respect to 100% attributed to the negative control sample.

Such result features a higher valence also considering the fact that the time 24 hours + 24 hours recovery is a particularly severe treatment because it allows the effects of delayed toxicity be observed.

In the absence of other toxicity elements at the histological level such increase can be correlated with a stimulation of the cellular proliferation that is induced by the treatment with the BIOMASS.

## 24 HOURS TREATMENTS

No meaningful modification in the tissues treated with the Biomass with respect to the negative control sample at 1%. At 3% and in the presence of the preserving agent it is observed that for some basal cells there is a cohesion loss and a cytoplasm slightly swollen (spongiosis).

## 24 HOURS TREATMENTS + 24 HOURS RECOVERY

No meaningful modification is observed with respect to the 24 hours control sample.

## 48 HOURS TREATMENTS

No meaningful morphological modification was observed with respect the control sample, increase in the epidermal thickness both at 1 % and at 3%.

In the presence of the preserving agent a slightly thickened horny layer is observed, with marks of surface flaking (S. disjunctum)

## 72 HOURS TREATMENTS

Both at 1 % and at 3% a decrease is observed of the vital epidermis thickness which is not meaningful with respect to the control sample which is in connection with the physiological evolution of the tissue.

## RT-PCR

Following a treatment with the Biomass at the various concentrations that were tested it is observed that already at 24 hours the action of stimulation to the expression of beta-defensin that goes on without exhausting with time till 72 hours where, anyway, it is of double value with respect to the control sample (PBS). This result corresponds to a meaningful stimulation to an increase in the physiological antimicrobial protection.

The presence of the antimicrobial preserving agent affects the study parameters globally with a reduction of the absolute values but the behavior of the Biomass keeps just the same; induction of the expression of beta-defensin 2.

## MEASUREMENT OF TEER

The values of TEER refer to an average of two tissues on which two measurements are carried out.

As to the value of TEER, the following items are relevant: the presence of a compact lamellar structure at the level of the horny layer, of integral tight junctions and the epidermis thickness that define globally an efficient barrier function. Each tissue is the proper reference with measurements at t = 0 and at t = a finite time.

At 24 hours + 24 hours recovery both in tissues treated and in the PBS = untreated control sampleof the lot a meaningless increase in the TEER is remarked.

The 48 hours result is particularly interesting, to which the most interesting histo-logies correspond (increase in the epidermal thickness) after treatment with the Biomass.

At 1% concentration a meaningful increase in the TEER is observed, as also occurs at 3% even though the TEER value is less sharp because of a high standard deviation.

This increase is a direct consequence both of the increase in the epidermal thickness and of the better compactness and integrality of the horny layer at the level of tight junctions.

At 72 hours no effect on the parameter is put into evidence, and the trend is anyway toward a decrease in the TEER after treatment, said decrease being partly correlated to the decrease in the thickness which is observed in histologies.

## GLUTAMATE-CYSTEINYL LIGASE

The GCLC is the enzymic precursor of the GSH synthesis: its expression has been studied starting from premature times after application (8H) as the redox equilibrium is the first epidermal mechanism of antioxidant defense.

No gene modulation under the experimental conditions chosen has been evidenced.

## GLUTATIONE SYNTHETASE

GSS is the enzzyme that limits the synthesis of antioxidant hydrophilic non-enzymic glutatione which is mainly present at the epidermal level.

The gene modulation is observed just as a tendency at 24 hours both for GABA and for the Biomass, but the expression values are very low and meaningless. Anyway it is important to stress that the meningful sub-expression at premature times is indirectly a positive signal as to the compatibility of the product and its physiological action at the dermal or the epidermal level: in the absence of external stimuli (for instance, induced oxidative stress) the physiological redox equilibrium which doesn't ask for an increase in the GSH synthesis is kept.

**HYALURONATE SYNTHETASE**

Thw HAS is the enzyme put in charge of the synthesis of hyaluronic acid, said synthesis being carried out by fibroblasts at the dermal level; it is important to stress the fact that the stimulation of fibroblast activity in the absence of negative stimuli is important for the expected efficiency on healthy skin and for everyday use.

Its expression is increased by the GABA action at 24 and 48 hours, as is disclosed in the literature and with a higher evidence by the treatment of the Biomass at 24 hours where an expression of triple vaue with respect to the control sample (figure 9).

The high standard deviation requires a further test for checking the data obtained.

PHILAGRIN

Prophilagrin, the precursor of 400-kDA of philagrin is the main component of keratoahyalin granules in human epidermis.

After the terminal differentiation in the horny layer, prophilagrin is transformed into philagrin, a protein of 37kD.

Philagrin contributes to aggregating and compacting the keratin in the cytoskeleton, and brings about the formation of the horny invlucre, which is the most external part of human body.

Marker of the epidermal differentiation, philagrin is considered to be a key protein for the barrier function and a fundamental element in the formation of NMF.

After distruction of that barrier, a loss of calcium and a decrease in the mRNA levels of philagrin occur.

Both treatment with GABA and with BIOMASS induce an over-expession of the gene that is evident for the GABA already at 24 hours and is significative for both starting from the 72 hours time; the Biomass has induced the gene expression higher than the GABA (Figure 10).

**INVOLUCRIN**

A cytoplasmic protein, soluble, of 68 kD and marker of the terminal differentiation of kerato-cytes.

It is the substrate of transglutaminase in keratinocytes and works as a 'scaffold' for the formation of the horny involucre in epidermis.

The gene expression starts in the thorny layer and is kept in the granulous layer.

After distruction of the barrier a loss of calcium and a decrease in the levels of mRNA of involucrin occur.

It works as a 'scaffold' also for the lipids (ceramides, cholesterol and fatty acids).

Globally no significative modulation of the gene occurs under the experimental conditions chosen (Fig. 11).

We proceeded to confirm what we had observed on the FT-skin with respect to the expression of philagrin and involucrin also on the basis of its epidermal model (RHE) for the 1% BIOMASS; results confirm a significative modulation for philagrin and an insignificant modulation for involucrin.

**CONCLUSIONS**

This research work, realized with the object of proving the efficiency of Biomass from Lactobacillus plantarum on different cutaneous mechanisms at the epidermal and the dermal level, has shown:

**THE IRRITATING POTENTIAL ON RHE:** the tolerability of the compound Biomass applied at 1% and 3% for times from 24 to 72 hours is total, without any indication of toxicity, evidenced both by means of cellular (cytotoxicity) and by morphological (H&E) parameters, at the level of the vital tissue.

The BIOMASS has induced a cellular proliferation stimulus both at 1% and at 3% on the basis of the data of cellular vitality higher than the negative control sample, of the increase in the vital epidermis thickness and of the expression of beta-defensin-2 that in addition suggests a physiological reaction of stimulation to anti-microbial defense.

**ACTION ON THE BARRIER FUNCTION ON RHE:** The result confirm the recognized GABA function as the measurement of the TEER points out a significative 48 hours increase in the TEER and to which the most interesting histologies correspond (increase in the epidermal thickness) after treatment with the Biomass both at 1% and at 3%. The result is the direct consequence both of the increase in the epidermal thickness and of the better compactness and integrality of the horny layer at the level of tight junctions.

**ACTION ON THE SYNTHESIS OF HYALURONIC ACID AND ON THE REDOX EQUILIBRIUM** on the FT-skin model with respect to the gamma-aminobutyric acid (GABA)

Following repeated applications for 3 days of the 1% Biomass the significative over-expression of the hyaluronate synthetase has been observed, in an amount higher than the values obtained with the GABA. The value of this data is connected to the fact that it confirms the literature on GABA and defines the Biomass as a product that when applied topically exerts a premature constant stimulation action of the fibroblasts to the neo-synthesis of hyaluronic acid.

The physiological stimulation action to the synthesis of hyaluronic acid hypothesizes a dermatological result of:

a larger turgidness of derma correlated with a higher capability of derma fibres to keep water at a deep level
a better cutaneous elasticity with time because the elastic fibres and collagen fibres are wrapped within a matrix that is characteristic of young and elastic skin (i.e., a skin rich of deep water).

**ACTION ON THE MARKERS OF TERMINAL DIFFERENTIATION:** philagrin and involucrin monitored on the FT-skin model: after repeated application for 3 days the involucrin was shown to be unmodulated, while the significative increase in the philagrin expression induced both by the Biomass and by GABA was observed. Such results puts into evidence the efficiency of the Biomass in relaunching the production mechanisms of the natural moisturizing factor NMF synthesized at the level of the granular layer.

**Claims**

1. *Lactobacillus plantarum* DSM 19463 or *Lactococcus lactis* ssp. DSM 19464 or their associations.

2. Use of *Lactobacillus plantarum* DSM 19463 or of *Lactococcus lactis* ssp. DSM 19464 or their associations for the preparation of a probiotic mixture enriched with gamma-aminobutyric acid.

3. Process for the preparation of a probiotic mixture enriched with gamma-aminobutyric acid comprising the following steps:

    a) propagation on a culture medium of *Lactobacillus plantarum* DSM 19463 or of *Lactococcus lactis* ssp. DSM 19464 for 24 hours at 30°C;
    b) resuspension of the biomass obtained in step a) in an aliquot of substrate added with monosodic L-glutamate 20 mM, said substrate being must according to the cell density of about $10^9$ ufc/g;
    c) inoculation of the substrates at 1-4% and 72-96 fermentation hours, preferably 72-78 fermentation hours, at a temperature from 30 to 33°C, preferably at 30°C.

4. Process according to claim 3, wherein the culture medium of step a) is MRS or m17.

5. Process according to anyone of the claims 3-4, wherein must is diluted with yeast water or is added with an extract of yeast.

6. Process according to anyone of the claims 3-5, wherein must is diluted with yeast water and with distilled water 50:50 ad it is deacidified by means of NaOH 1 N at a pH value from 6,0 to 6,5, preferably at a pH of 6,0.

7. Process according to anyone of the claims 3-6, wherein the must is added with pyridoxal 5-phosphate.

8. Process according to anyone of the claims 3-6, which further comprises a step d) of desiccation or lyophilization of the fermentation substrate that is obtained from the step c).

9. Mixture enriched with gamma-aminobutyric acid by means of a process according to anyone of claims 3-8, said mixture comprising *Lactobacillus plantarum* DSM 19463 or *Lactococcus lactis* ssp. DSM 19464 or their associations.

10. Mixture enriched with gamma-aminobutyric acid according to claim 9, which comprises at least 890 mg of gamma-aminobutyric acid per 100 g of dry substance.

11. Mixture enriched with gamma-aminobutyric acid according to anyone of claims 9-10, which further comprises vitamins, minerals, polyphenols and alive and vital lactic bacteria.

12. Pharmaceutical or cosmetic composition comprising a mixture enriched with $\gamma$-aminobutyric acid according to anyone of claims 9-11, as the active principle, together with one or more pharmaceutically or cosmetically acceptable excipients and/or adjuvants.

13. Use of the mixture enriched with $\gamma$-aminobutyric acid according to anyone of claims 9-11 as a cosmetic.

14. Use of the mixture enriched with gamma-aminobutyric acid according to anyone of claims 9 - 11 or having the composition according to claim 12 for the preparation of a medicament for dermatological use.

15. Pharmaceutical composition according to claim 12 for use in the treatment of renal damages.

**Patentansprüche**

1. *Lactobacillus plantarum* DSM 19463 oder *Lactococcus lactis* ssp. DSM 19464 oder deren Assoziationen.

2. Verwendung von *Lactobacillus plantarum* DSM 19463 oder *Lactococcus lactis* ssp. DSM 19464 oder deren Assoziationen zur Herstellung einer probiotischen Mischung, die mit gamma-Aminobuttersäure angereichert ist.

3. Verfahren zur Herstellung einer probiotischen Mischung, die mit gamma-Aminobuttersäure angereichert ist, umfassend die folgenden Schritte:

    a) Propagieren eines Kulturmediums von *Lactobacillus plantarum* DSM 19463 oder *Lactococcus lactis* ssp. DSM 19464 für 24 h bei 30 °C;

b) Resuspendieren der in Schritt a) erhaltenen Biomasse in einem Aliquot von Substrat, welches mit L-Mononatriumglutamat 20 mM versetzt ist, wobei das Substrat Molke (engl. *must*) gemäß einer Zelldichte von ungefähr $10^9$ ufc/g ist;
c) Animpfen der Substrate bei 1 - 4 % und 72 - 96 Fermentationsstunden, bevorzugt 72 - 78 Fermentationsstunden, bei einer Temperatur von 30 bis 33 °C, bevorzugt 30 °C.

**4.** Verfahren nach Anspruch 3, wobei das Kulturmedium des Schritts a) M RS oder m 17 ist.

**5.** Verfahren nach einem der Ansprüche 3 - 4, wobei die Molke mit Hefewasser verdünnt wird oder mit einem Hefeextrakt versetzt wird.

**6.** Verfahren nach einem der Ansprüche 3 - 5, wobei die Molke mit Hefewasser und mit destilliertem Wasser 50:50 verdünnt wird und mittels NaOH 1 N auf einen pH von 6,0 bis 6,5, bevorzugt auf einen pH von 6,0, entsäuert wird.

**7.** Verfahren nach einem der Ansprüche 3 - 6, wobei die Molke mit Pyridoxal-5-phosphat versetzt wird.

**8.** Verfahren nach einem der Ansprüche 3 - 6, welches ferner einen Schritt d) der Trocknung oder Lyophilisierung des Fermentationssubstrats, welches in Schritt c) erhalten wird, umfasst.

**9.** Mischung angereichert mit gamma- Aminobuttersäure mittels eines Verfahrens nach einem der Ansprüche 3 - 8, wobei die Mischung *Lactobacillus plantarum* DSM 19463 oder *Lactococcus lactis* ssp. DSM 19464 oder deren Assoziationen umfasst.

**10.** Mischung angereichert mit gamma-Aminobuttersäure nach Anspruch 9, welche wenigstens 890 mg gamma-Aminobuttersäure pro 100 g Trockensubstanz umfasst.

**11.** Mischung angereichert mit gamma-Aminobuttersäure nach einem der Ansprüche 9 - 10, welche ferner Vitamine, Mineralien, Polyphenole und lebende und vitale Milchsäurebakterien umfasst.

**12.** Pharmazeutische oder kosmetische Zusammensetzung umfassend eine mit $\gamma$-Aminobuttersäure angereicherte Mischung nach einem der Ansprüche 9 - 11 als Wirkstoff zusammen mit einem oder mehreren pharmazeutisch oder kosmetisch verträglichen Hilfsstoffen und/oder Zusatzstoffen.

**13.** Verwendung der mit $\gamma$-Aminobuttersäure angereicherten Mischung nach einem der Ansprüche 9 - 11 als Kosmetikum.

**14.** Verwendung der mit $\gamma$-Aminobuttersäure angereicherten Mischung nach einem der Ansprüche 9 - 11 oder einer, die die Zusammensetzung nach Anspruch 12 aufweist, zur Herstellung eines Medikaments zur dermatologischen Verwendung.

**15.** Pharmazeutische Zusammensetzung nach Anspruch 12 zur Verwendung in der Behandlung von Nierenschäden.

**Revendications**

**1.** *Lactobacillus plantarum* DSM 19463 ou *Lactococcus lactis* ssp. DSM 19464 ou leurs associations.

**2.** Utilisation de *Lactobacillus plantarum* DSM 19463 ou de *Lactococcus lactis* ssp. DSM 19464 ou de leurs associations pour la préparation d'un mélange probiotique enrichi en acide gamma-aminobutyrique.

**3.** Procédé de préparation d'un mélange probiotique enrichi en acide gamma-aminobutyrique comprenant les étapes suivantes :

a) propagation sur un milieu de culture de *Lactobacillus plantarum* DSM 19463 ou de *Lactococcus lactis* ssp. DSM 19464 pendant 24 heures à 30 °C ;
b) remise en suspension de la biomasse obtenue à l'étape a) dans un aliquote de substrat auquel a été ajouté du L-glutamate monosodique 20 mM, ledit substrat étant du moût conformément à la densité cellulaire d'environ $10^9$ ufc/g ;
c) inoculation des substrats à 1 à 4 % et 72 à 96 heures de fermentation, de préférence 72 à 78 heures de fermentation, à une température de 30 à 33 °C, de préférence de 30 °C.

4. Procédé selon la revendication 3, dans lequel le milieu de culture de l'étape a) est le MRS ou le m17.

5. Procédé selon l'une quelconque des revendications 3 à 4, dans lequel le moût est dilué avec de l'eau de levure ou dans lequel un extrait de levure est ajouté au moût.

6. Procédé selon l'une quelconque des revendications 3 à 5, dans lequel le moût est dilué avec de l'eau de levure et avec de l'eau distillée 50 :50 puis est désacidifié au moyen de NaOH 1 N à une valeur de pH de 6,0 à 6,5, de préférence à un pH de 6,0.

7. Procédé selon l'une quelconque des revendications 3 à 6, dans lequel du 5-phosphate de pyridoxal est ajouté au moût.

8. Procédé selon l'une quelconque des revendications 3 à 6, qui comprend en outre une étape d) de dessiccation ou de lyophilisation du substrat de fermentation qui est obtenu à l'étape c).

9. Mélange enrichi en acide gamma-aminobutyrique au moyen d'un procédé selon l'une quelconque des revendications 3 à 8, ledit mélange comprenant *Lactobacillus plantarum* DSM 19463 ou *Lactococcus lactis* ssp. DSM 19464 ou leurs associations.

10. Mélange enrichi en acide gamma-aminobutyrique selon la revendication 9, qui comprend au moins 890 mg d'acide gamma-aminobutyrique pour 100 g de substance sèche.

11. Mélange enrichi en acide gamma-aminobutyrique selon l'une quelconque des revendications 9 à 10, qui comprend en outre des vitamines, des minéraux, des polyphénols et des bactéries lactiques vivantes et vitales.

12. Composition pharmaceutique ou cosmétique comprenant un mélange enrichi en acide $\gamma$-aminobutyrique selon l'une quelconque des revendications 9 à 11, en tant que principe actif, conjointement à un ou plusieurs excipients et/ou adjuvants pharmaceutiquement ou cosmétiquement acceptables.

13. Utilisation du mélange enrichi en acide $\gamma$-aminobutyrique selon l'une quelconque des revendications 9 à 11 en tant que produit cosmétique.

14. Utilisation du mélange enrichi en acide $\gamma$-aminobutyrique selon l'une quelconque des revendications 9 à 11 ou ayant la composition selon la revendication 12, pour la préparation d'un médicament pour une utilisation dermatologique.

15. Composition pharmaceutique selon la revendication 12, pour son utilisation dans le traitement des lésions rénales.

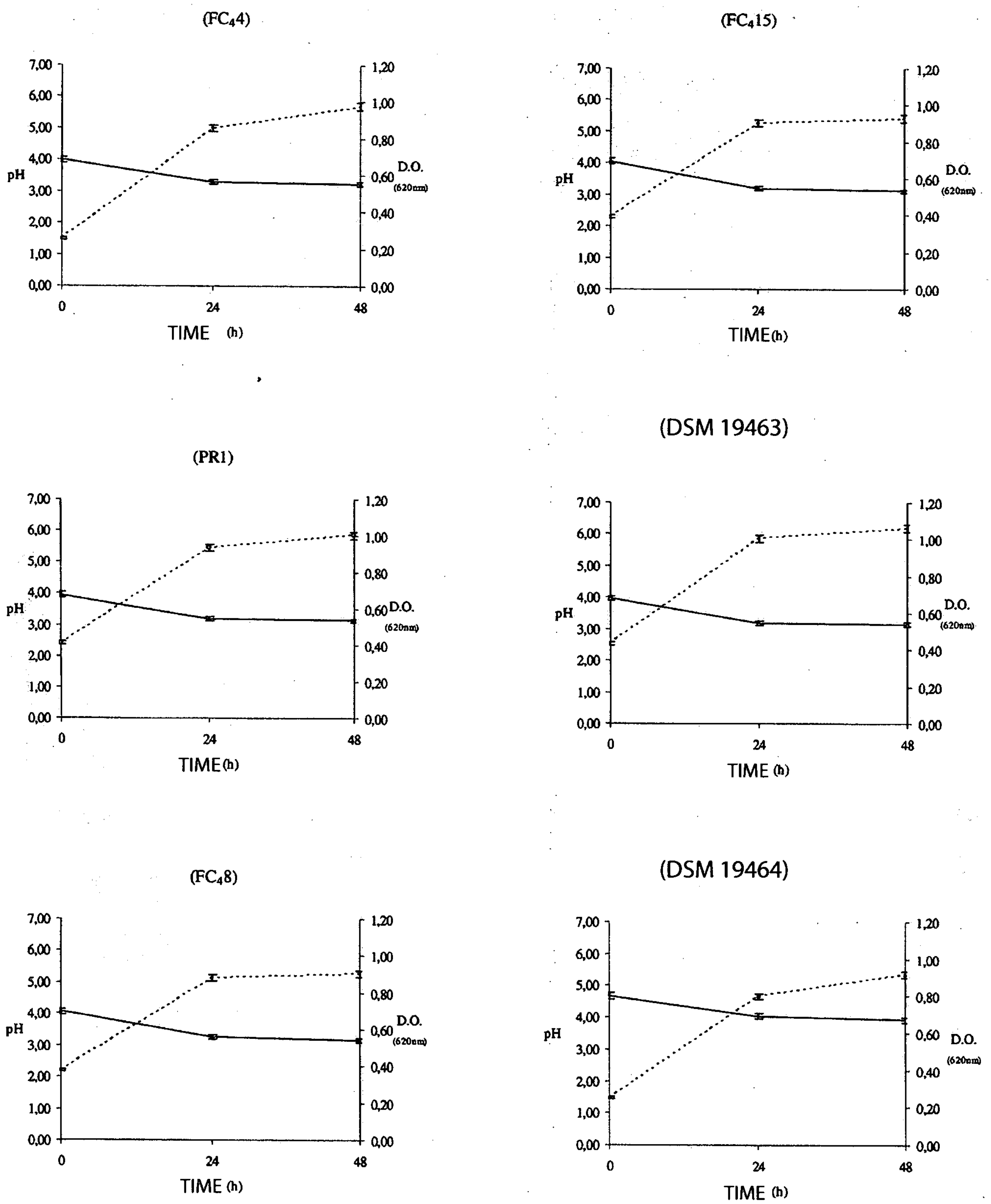
(FC44)
(FC415)
(PR1)
(DSM 19463)
(FC48)
(DSM 19464)
pH
D.O. (620nm)
TIME (h)
7,00
6,00
5,00
4,00
3,00
2,00
1,00
0,00
1,20
1,00
0,80
0,60
0,40
0,20
0,00
0
24
48

**Fig. 1**

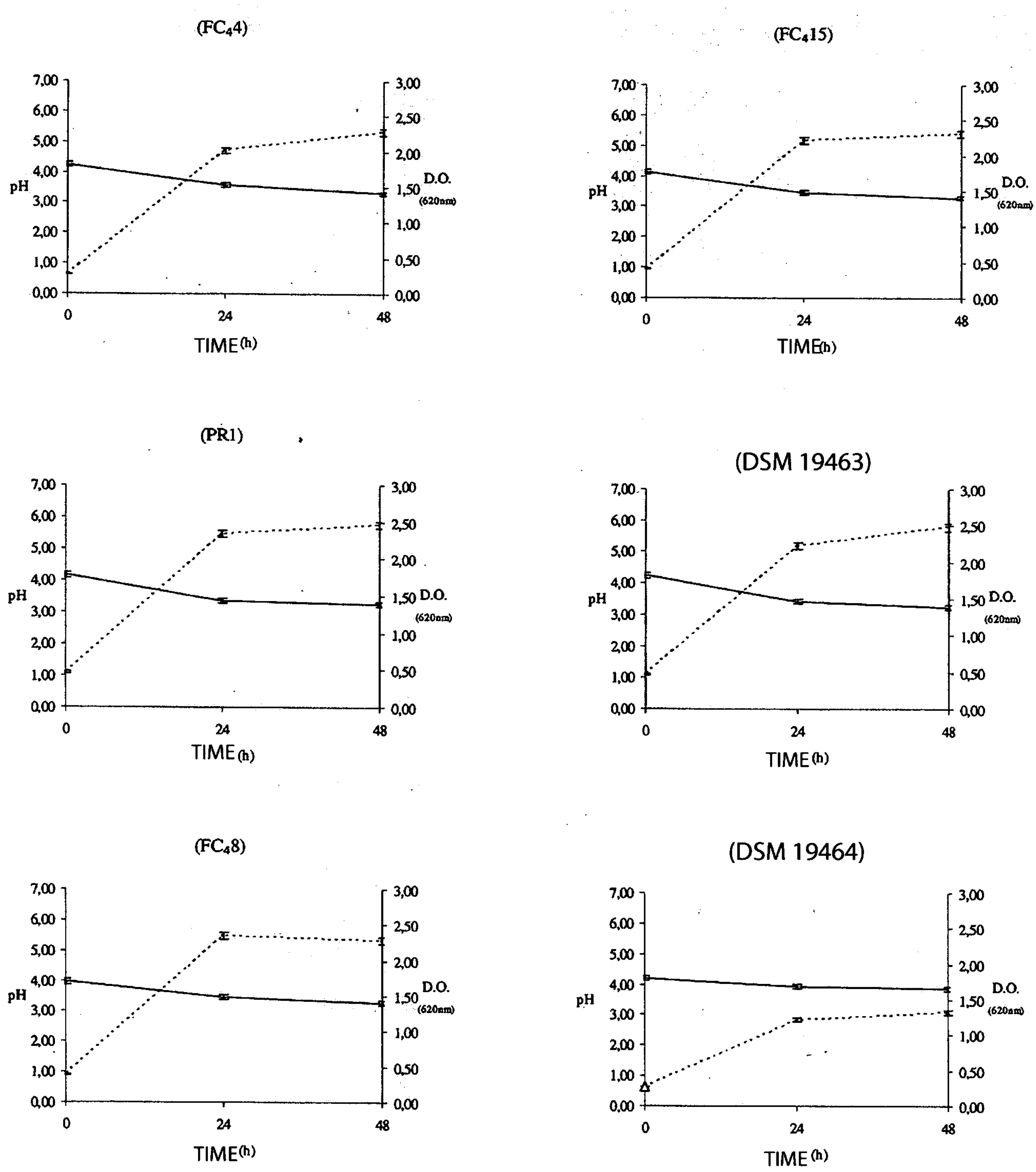
(FC4 4)
(FC4 15)
(PR1)
(DSM 19463)
(FC4 8)
(DSM 19464)
pH
D.O. (620nm)
TIME (h)
7,00
6,00
5,00
4,00
3,00
2,00
1,00
0,00
3,00
2,50
2,00
1,50
1,00
0,50
0,00
0
24
48

*Fig. 2*

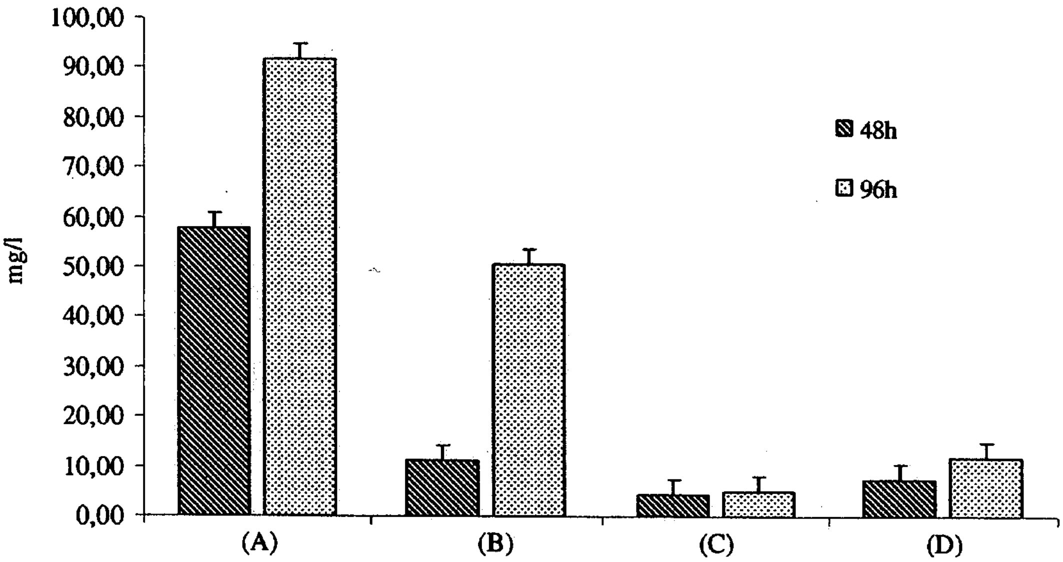
100,00
90,00
80,00
70,00
60,00
50,00
40,00
30,00
20,00
10,00
0,00
mg/l
48h
96h
(A)
(B)
(C)
(D)

*Fig. 3*

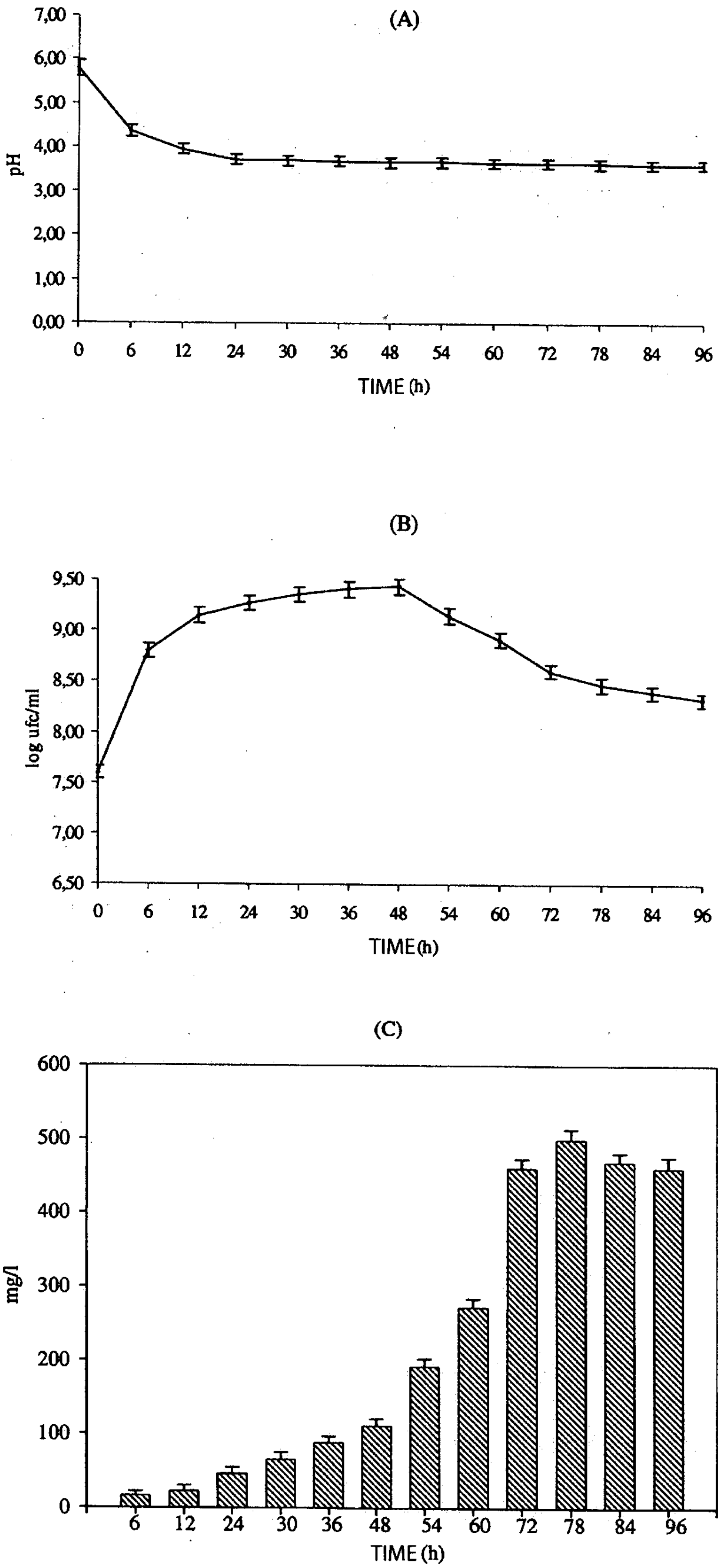
(A)
7,00
6,00
5,00
4,00
3,00
2,00
1,00
0,00
pH
0
6
12
24
30
36
48
54
60
72
78
84
96
TIME (h)
(B)
9,50
9,00
8,50
8,00
7,50
7,00
6,50
log ufc/ml
0
6
12
24
30
36
48
54
60
72
78
84
96
TIME (h)
(C)
600
500
400
300
200
100
0
mg/l
6
12
24
30
36
48
54
60
72
78
84
96
TIME (h)

*Fig. 4*

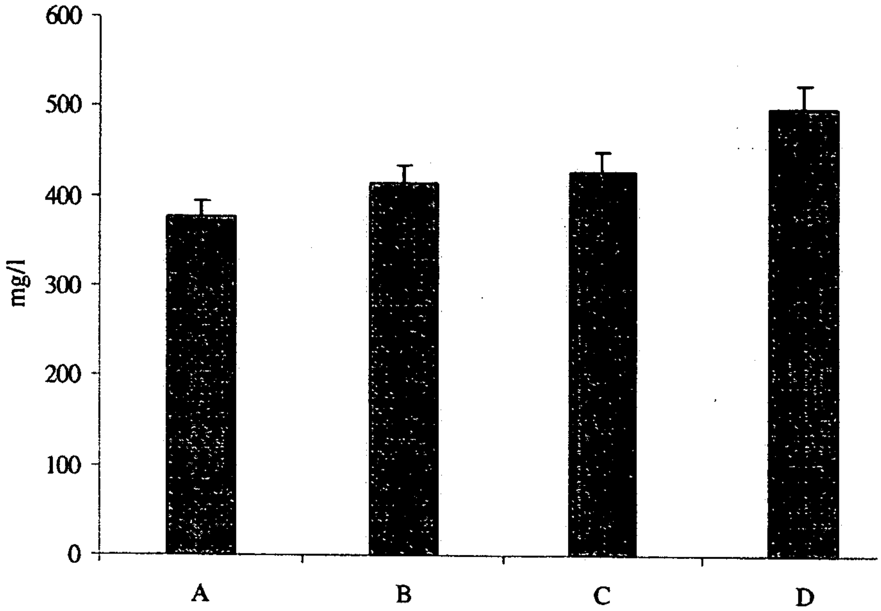


Fig. 5

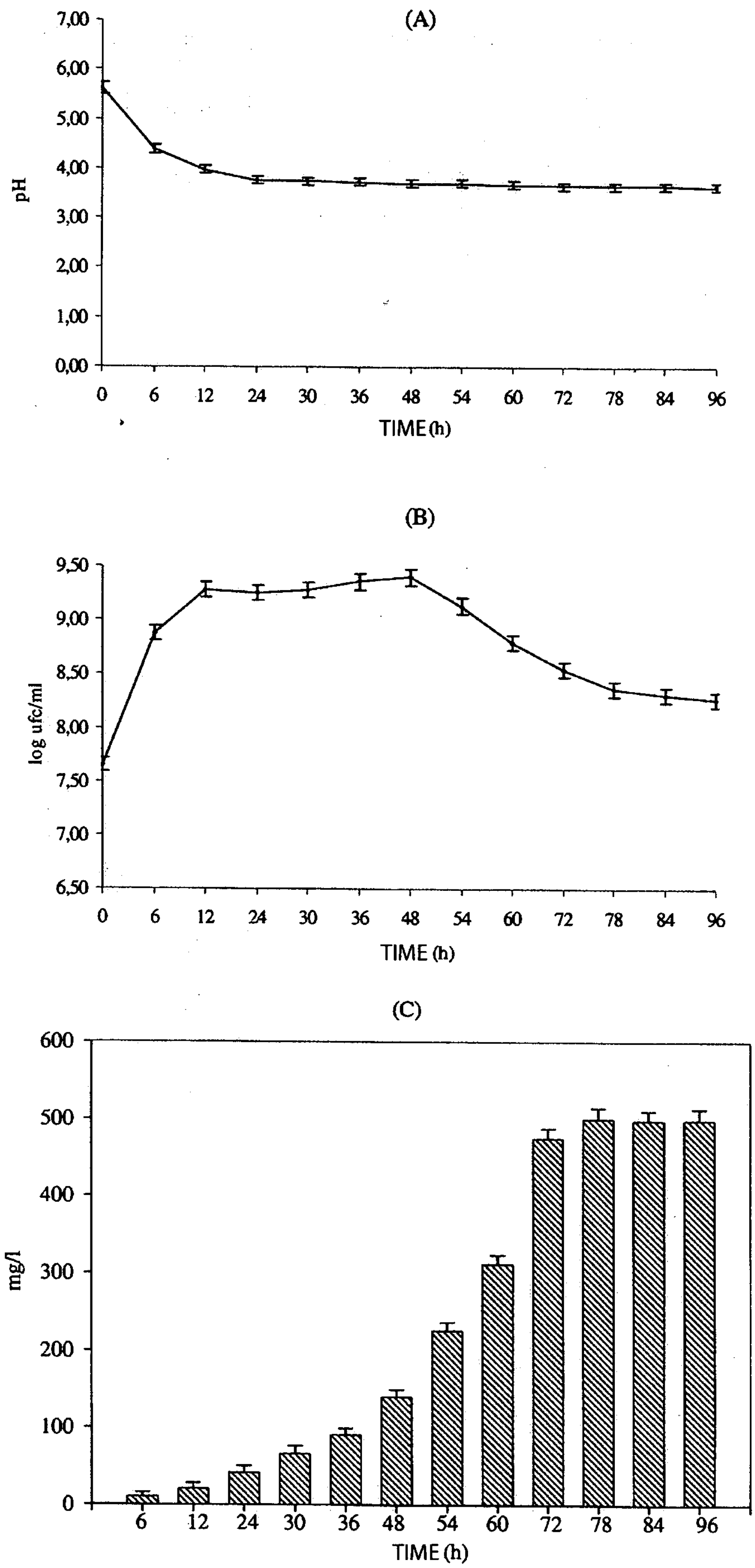
(A)
pH
7,00
6,00
5,00
4,00
3,00
2,00
1,00
0,00
0 6 12 24 30 36 48 54 60 72 78 84 96
TIME (h)
(B)
log ufc/ml
9,50
9,00
8,50
8,00
7,50
7,00
6,50
0 6 12 24 30 36 48 54 60 72 78 84 96
TIME (h)
(C)
mg/l
600
500
400
300
200
100
0
6 12 24 30 36 48 54 60 72 78 84 96
TIME (h)

*Fig. 6*

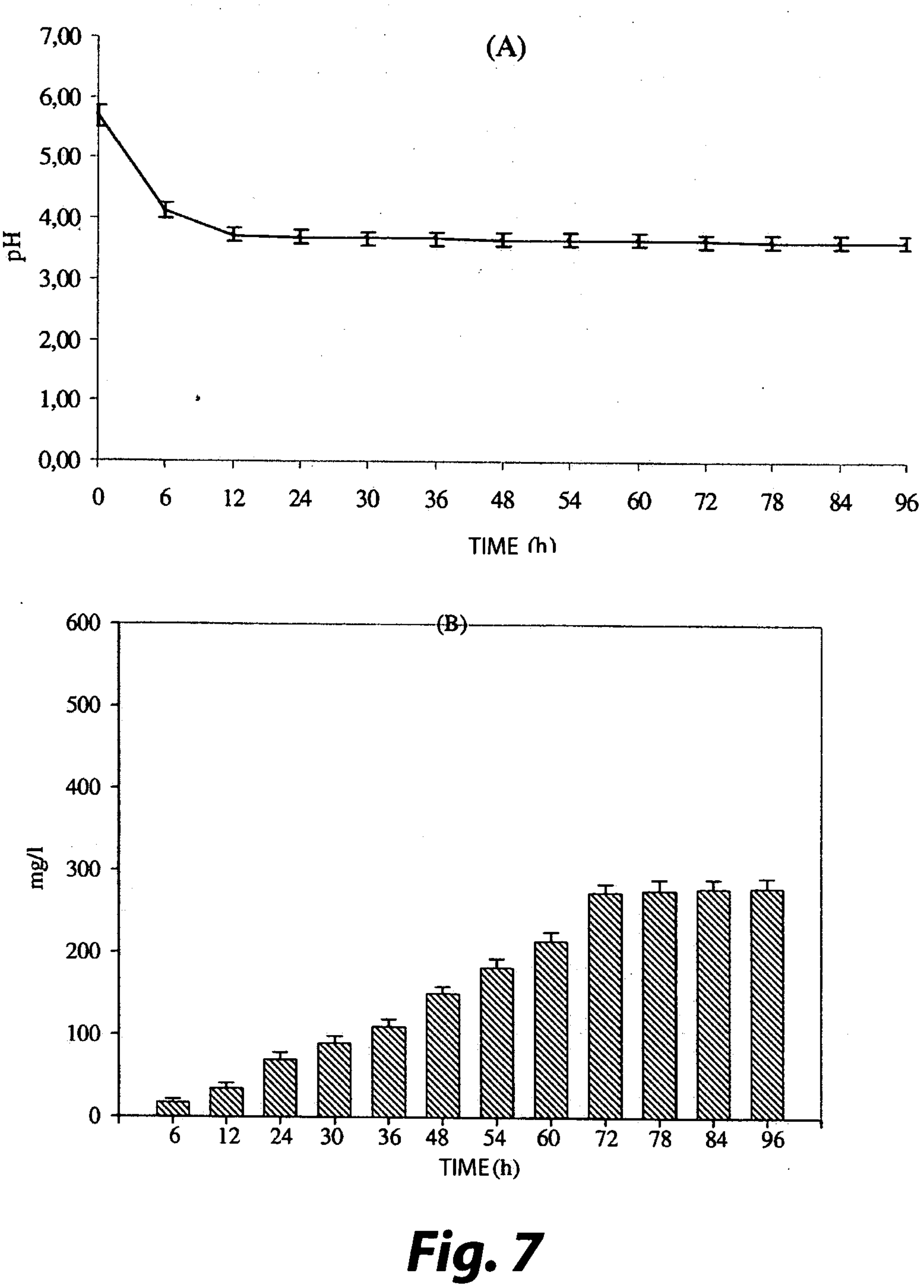
(A)
7,00
6,00
5,00
4,00
3,00
2,00
1,00
0,00
pH
0
6
12
24
30
36
48
54
60
72
78
84
96
TIME (h)
(B)
600
500
400
300
200
100
0
mg/l
6
12
24
30
36
48
54
60
72
78
84
96
TIME (h)

Fig. 7

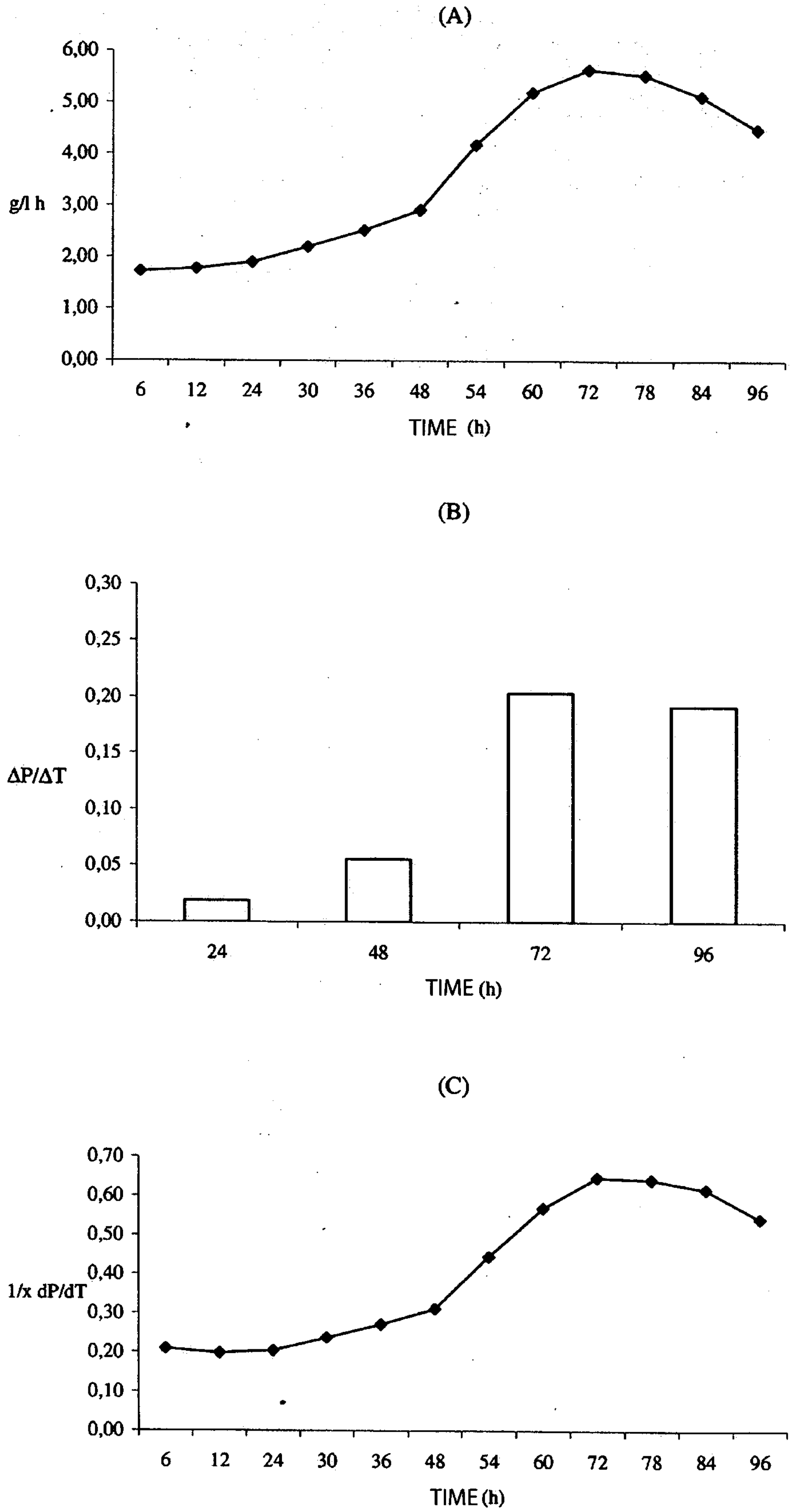
(A)
6,00
5,00
4,00
g/l h
3,00
2,00
1,00
0,00
6 12 24 30 36 48 54 60 72 78 84 96
TIME (h)
(B)
0,30
0,25
0,20
ΔP/ΔT
0,15
0,10
0,05
0,00
24 48 72 96
TIME (h)
(C)
0,70
0,60
0,50
0,40
1/x dP/dT
0,30
0,20
0,10
0,00
6 12 24 30 36 48 54 60 72 78 84 96
TIME (h)

Fig. 8

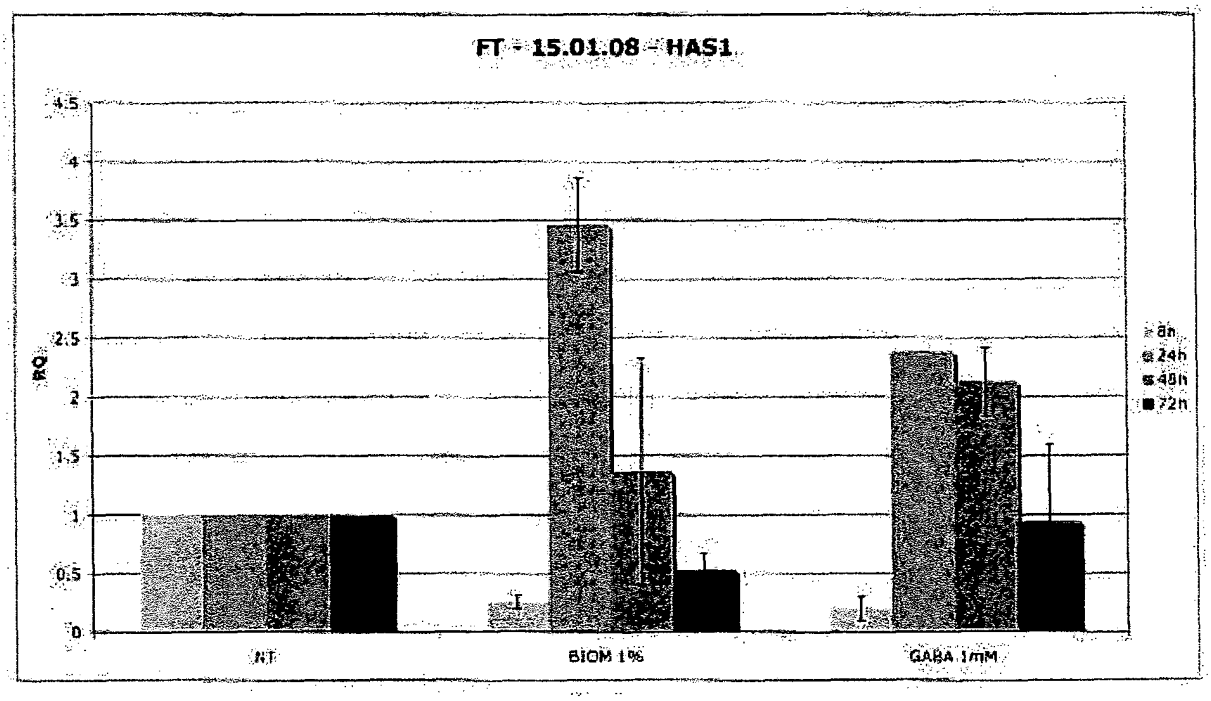
FT - 15.01.08 - HAS1
RQ
4.5
4
3.5
3
2.5
2
1.5
1
0.5
0
8h
24h
48h
72h
NT
BIOM 1%
GABA 1mM

*Fig. 9*

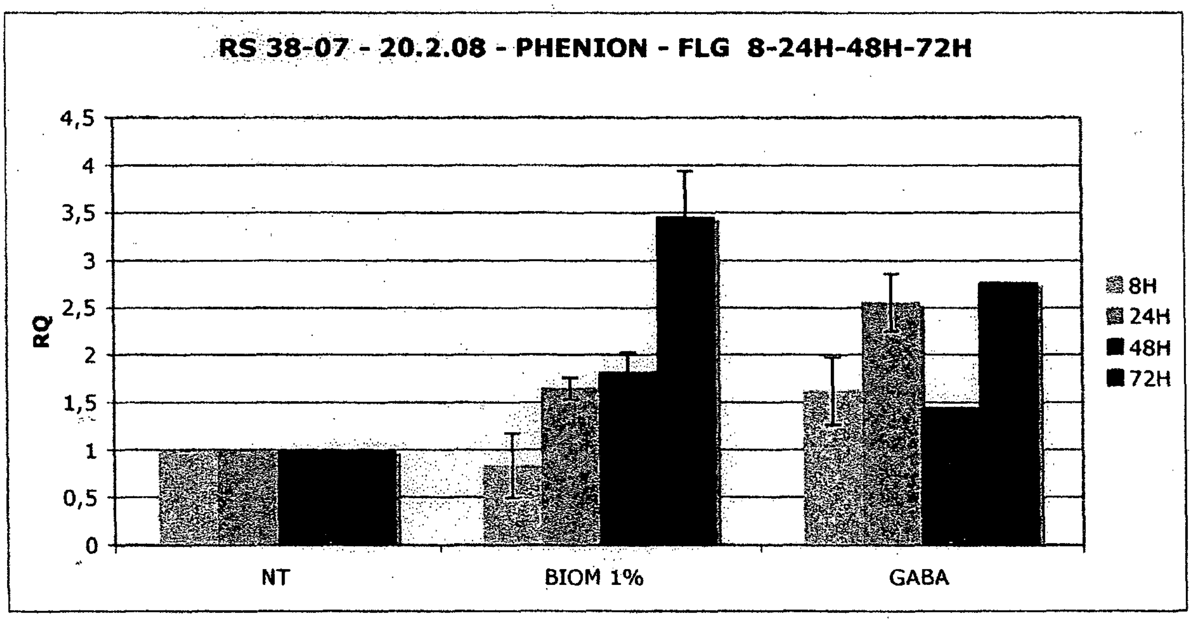
RS 38-07 - 20.2.08 - PHENION - FLG 8-24H-48H-72H
RQ
4,5
4
3,5
3
2,5
2
1,5
1
0,5
0
8H
24H
48H
72H
NT
BIOM 1%
GABA

*Fig. 10*

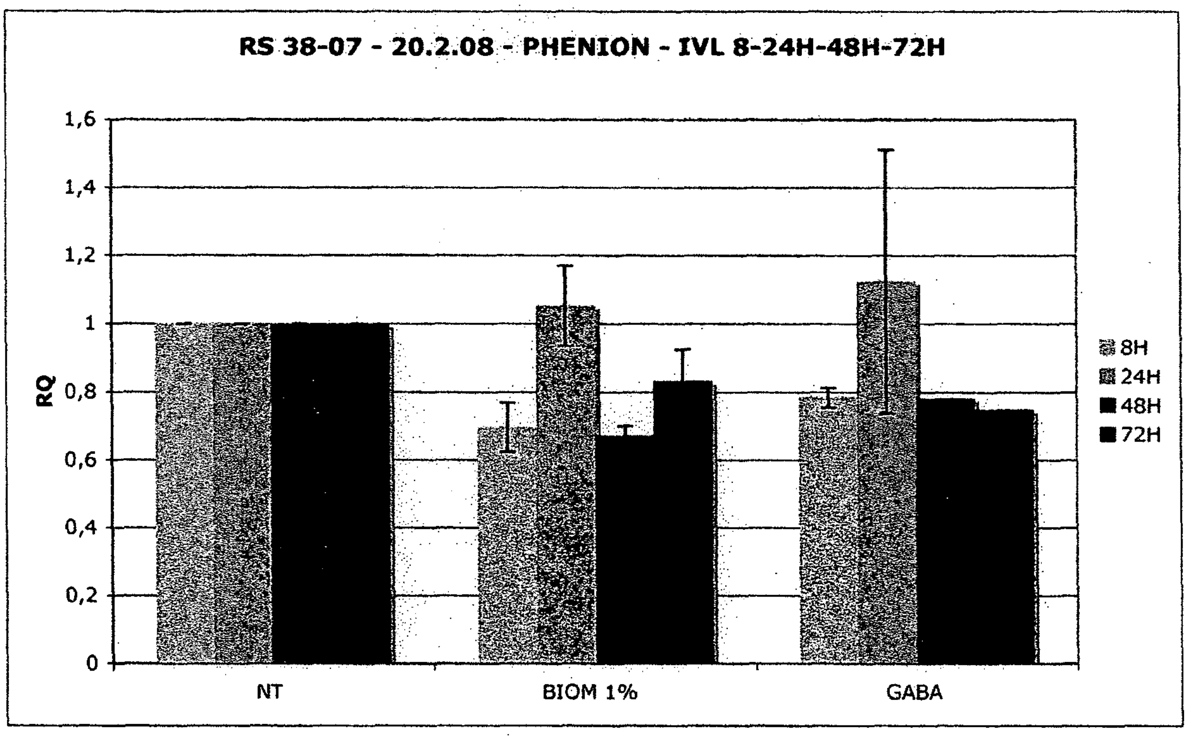
RS 38-07 - 20.2.08 - PHENION - IVL 8-24H-48H-72H
1,6
1,4
1,2
1
0,8
0,6
0,4
0,2
0
RQ
NT
BIOM 1%
GABA
8H
24H
48H
72H

*Fig. 11*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005102559 A
- JP 2002360289 B
- WO 2007075011 A


### Non-patent literature cited in the description


- **UENO.** Enzymatic and structural aspect on decarboxylase. *J. Mol. Catal.,* 2000, vol. 10, 67-79
- **GUIN et al.** GABA, gamma-hydroxybutyric acid, and neurological disease. *Ann. Neur.,* 2003, vol. 6, 3-12
- **JAKOBS et al.** Inherited disorders of GABA metabolism. *J. Inherit. Metab.Di,* 1993, vol. 16, 704-715
- **OKADA et al.** Effect of the defatted rice germ enriched with GABA for sleeplessness, depression, autonomic disorder by oral administration. *Nippon Shokuhin Kagaku Kaishi,* 2000, vol. 75, 596-603
- **OH et al.** Germinated brown rice extract shows a nutraceutical effect in the recovery of chronic alcohol-related symptoms. *J. Med. Food.,* 2003, vol. 6, 115-121
- **OH.** Brown rice extracts with enhanced levels of GABA stimulate immune cells. *Food Sci. Biotechnol.,* 2003, vol. 12, 248-252
- **WARSKULAT et al.** The osmolyte strategy of normal human keratinocytes in mantaining cell homeostasis. *J. Invest. Dermatol.,* 2004, vol. 123, 516-521
- **DENDA et al.** gamma-aminobutyric aid (A)receptor agonists accelerate cutaneous barrier recovery and prevent epidermal hyperplasia induced by barrier disruption. *J. Invest. Dermatol.,* 2002, vol. 119, 1041-1047
- **ITO et al.** GABA-synthesizing enzyme, GAD67, from dermal fibroblasts: evidence for a new skin function. *Biochim. Biophys. Acta,* vol. 1770, 291-296
- **SATINDER S. et al.** *The Journal of pharmacology and experimental therapeutics,* 2008, vol. 324 (1), 376-382
- **NAGAOKA.** Treatment of germinated wheat to increase levels of GABA and IP6 catalyzed by endogenous ezymes. *Biotechnol. Prog.,* 2005, vol. 21, 405-410
- **OH.** Stimulation of gamma-aminobutyric acid synthesis activity in brown rice by a chitosan/Glu germination solution and calcium/calmodulin. *J. Biochem. Mol. Biol.,* 2003, vol. 36, 319-325
- **NOMURA et al.** Production of gamma-aminobutyric acid by cheese starters during cheese ripening. *J. Dairy Sci.,* 1998, vol. 81, 1486-1491
- **PARK ; OH.** Cloning and expression of a full length glutamate decarboxylase gene from Lactobacillus plantarum. *J. Food Sci. Nutr.,* 2004, vol. 9, 324-329
- **PARK ; OH.** Cloning, sequencing and expression of a novel glutamate decarboxylase gene from a newly isolated lactic acid bacterium. *Lactobacillus brevis OPK-3, Bioresour. Technol.,* 2007, vol. 98, 312-319
- **COTTER ; HILL.** Surviving the acid text: responses of the of Gram-positive bacteria to low pH. *Microbiol. MoLRev.,* 2003, vol. 67, 429-453
- **HIGUCHI et al.** Exchange of glutamate and gamma-aminobutyrate in a Lactobacillus strain. *J. Bacteriol.,* 1997, vol. 179, 3362-3364
- **KOMATSUZAKI et al.** Production of gamma-aminobutyric acid (GABA) by Lactobacillus paracasei isolated from traditional fermented foodstuffs. *Food Microbiol,* 2005, vol. 22, 497-504
- **ZWIETERING et al.** Modelling of bacterial growth curve. *Appl. Environ. Microbiol.,* 1990, vol. 56, 1875-1881
- **DI CAGNO et al.** Characterization of Italian Cheeses Ripened Under Nonconventional Conditions. *J. Dairy Sci.,* 2007, vol. 90, 2689-2704
- **DE LOS REYES-GAVILAN et al.** A Lactobacillus helveticus-specific DNA probe detects restriction fragment length polymorphisms in this species. *Appl. Environ. Microbiol.,* vol. 58, 3492-3432